# EUROPEAN PATENT SPECIFICATION

(11) **EP 1 250 435 B1**
(45) Date of publication and mention of the grant of the patent: **12.04.2006**
(21) Application number: 01926593.3
(22) Date of filing: 26.01.2001
(51) Int. Cl.: C12N 15/12, C12N 15/11, C12N 15/62, C07K 14/05, C07K 16/28, C12Q 1/68, A61K 38/00

(54) **HUMAN NEUREXIN-LIKE PROTEINS AND POLYNUCLEOTIDES ENCODING THE SAME**
MENSCHLICHE NEUREXIN-ÄHNLICHE PROTEINE UND DAFÜR KODIERENDE POLYNUKLEOTIDE
PROTEINES HUMAINES DU TYPE NEUREXINE ET POLYNUCLEOTIDES CODANT POUR CELLES-CI

(30) Priority: 26.01.2000 US 178557 P; 25.04.2000 US 199513 P
(43) Date of publication of application: 23.10.2002
(73) Proprietor: Lexicon Genetics Incorporated, The Woodlands, TX 77381-1160 (US)
(72) Inventor: TURNER, C., Alexander, Jr., The Woodlands, TX 77382 (US); HILBUN, Erin, Denton, TX 76209 (US); DONOHO, Gregory, Indianapolis, IN 46259 (US); SCOVILLE, John, Pearland , TX 77584 (US); WATTLER, Frank, 82541 Muensing (DE); FRIEDRICH, Glenn, c/o Breland & Breland, Houston, TX 77004 (US); ABUIN, Alejandro, The Woodlands, TX 77382 (US); ZAMBROWICZ, Brian, The Woodlands, TX 77382 (US); SANDS, Arthur, T., The Woodlands, TX 77382 (US)
(74) Representative: Silveston, Judith
(86) International application number: PCT/US2001/010815
(87) International publication number: WO 2001/058938

(56) References cited:
- WO-A-97/35872
- WO-A-99/53051
- DATABASE EM_EST [Online] EMBL Heidelberg, Germany; Accession number AA069426, 2 February 1997 (1997-02-02) HILLIER L ET AL.: "Homo sapiens cDNA clone IMAGE:382628" XP002184021 & HILLIER L ET AL.: GENOME RESEARCH, vol. 6, no. 9, 1996, pages 807-828,
- DATABASE EM_GSS [Online] EMBL Heidelberg, Germany; Acession number AQ635982, 17 June 1999 (1999-06-17) ZHAO S ET AL.: "Homo sapiens genomic clone RPCI-11-475A8" XP002184023
- DATABASE EM_HTG [Online] EMBL Heidelberg, Germany; Accession number AC015602, 20 September 2000 (2000-09-20) BIRREN B ET AL.: "Homo sapiens clone RP11-45D4" XP002184022
- DATABASE EM_EST [Online] EMBL Heidelberg, Germany; Accession number BF391472, 27 November 2000 (2000-11-27) BONALDO M F ET AL.: "Rattus norvegicus cDNA clone UI-R-CA1-bcs-c-10-0-UI" XP002184024 & BONALDO M F ET AL.: GENOME RESEARCH, vol. 6, no. 9, 1996, pages 791-806,

## Description

### 1. INTRODUCTION

The present invention relates to the discovery, identification, and characterization of novel human polynucleotides encoding proteins that share sequence similarity with animal neurexin proteins and contactin associated proteins. The invention encompasses the described polynucleotides, host cell expression systems, the encoded proteins, fusion proteins, polypeptides and peptides, antibodies to the encoded proteins and peptides, and genetically engineered animals that either lack or over express the disclosed sequences, antagonists and agonists of the proteins, and other compounds that modulate the expression or activity of the proteins encoded by the disclosed sequences that can be used for diagnosis, drug screening, clinical trial monitoring, the treatment of diseases and disorders, or cosmetic or nutriceutical applications.

### 2. BACKGROUND OF THE INVENTION

Neurexins have been associated with, *inter alia*, mediating neural processes, seizures, signaling, exocytosis, cancer, and development. Neurexins can also serve as receptors for latrotoxins.

### 3. SUMMARY OF THE INVENTION

The present invention relates to the discovery, identification, and characterization of nucleotides that encode novel human proteins and the corresponding amino acid sequences of these proteins. The novel human proteins (NHPs) described for the first time herein share structural similarity with neurexin proteins.

The novel human nucleic acid sequences described herein, encode alternative proteins/open reading frames (ORFs) of 1,307, 1,259, 35, 250, 279, 582, 534, 745, 697, 839, 791, 1,298, and 1,175 amino acids in length (see respectively SEQ ID NOS: 2, 4, 6, 8, 10, 12, 14, 16, 18, 20, 22, 24, and 26).

The invention also encompasses agonists and antagonists of the described NHPs, including small molecules, large molecules, mutant NHPs, or portions thereof that compete with native NHP, peptides, and antibodies, as well as nucleotide sequences that can be used to inhibit the expression of the described NHPs (e.g., antisense and ribozyme molecules, and gene or regulatory sequence replacement constructs) or to enhance the expression of the described NHP sequences (e.g., expression constructs that place the described sequence under the control of a strong promoter system), and transgenic animals that express a NHP transgene, or "knockouts" (which can be conditional) that do not express a functional NHP.

Further, the present invention also relates to processes for identifying compounds that modulate, i.e., act as agonists or antagonists, of NHP expression and/or NHP activity that utilize purified preparations of the described NHPs and/or NHP product, or cells expressing the same. Such compounds can be used as therapeutic agents for the treatment of any of a wide variety of symptoms associated with biological disorders or imbalances.

### 4. DESCRIPTION OF THE SEQUENCE LISTING AND FIGURES

The Sequence Listing provides the sequences of the described NHP ORFs that encode the described NHP amino acid sequences. SEQ ID NO:27 describes a NHP ORF and flanking regions.

### 5. DETAILED DESCRIPTION OF THE INVENTION

The NHPs, described for the first time herein, are novel proteins that are expressed in, *inter alia,* human cell lines, human fetal brain, brain, cerebellum, testis, adrenal gland, spinal cord, small intestine, hypothalamus, and gene trapped human cells.

The present invention encompasses the nucleotides presented in the Sequence Listing, host cells expressing such nucleotides, the expression products of such nucleotides, and: (a) nucleotides that encode mammalian homologs of the described sequences, including the described NHPs, and the NHP products;
(b) nucleotides that encode one or more portions of the NHPs that correspond to functional domains, and the polypeptide products specified by such nucleotide sequences, including but not limited to the novel regions of any active domain(s); (c) isolated nucleotides that encode mutant versions, engineered or naturally occurring, of the described NHPs in which all or a part of at least one domain is deleted or altered, and the polypeptide products specified by such nucleotide sequences, including but not limited to soluble proteins and peptides in which all or a portion of the signal sequence is deleted; (d) nucleotides that encode chimeric fusion proteins containing all or a portion of a coding region of a NHP, or one of its domains (*e.g*., a receptor or ligand binding domain, accessory protein/self-association domain, etc.) fused to another peptide or polypeptide; or (e) therapeutic or diagnostic derivatives of the described polynucleotides such as oligonucleotides, antisense polynucleotides, ribozymes, dsRNA, or gene therapy constructs comprising a sequence first disclosed in the Sequence Listing.

As discussed above, the present invention includes: (a) the human DNA sequences presented in the Sequence Listing (and vectors comprising the same) and additionally contemplates any nucleotide sequence encoding a contiguous NHP open reading frame (ORF) that hybridizes to a complement of a DNA sequence presented in the Sequence Listing under highly stringent conditions, *e.g*., hybridization to filter-bound DNA in 0.5 M NaHPO₄, 7% sodium dodecyl sulfate (SDS), 1 mM EDTA at 65°C, and washing in 0.1xSSC/0.1% SDS at 68°C (Ausubel F.M. et *al*., eds., 1989, Current Protocols in Molecular Biology, Vol. I, Green Publishing Associates, Inc., and John Wiley & sons, Inc., New York, at p. 2.10.3) and encodes a functionally equivalent gene product. Additionally contemplated are any nucleotide sequences that hybridize to the complement of a DNA sequence that encodes and expresses an amino acid sequence presented in the Sequence Listing under moderately stringent conditions, *e.g*., washing in 0.2xSSC/0.1% SDS at 42°C (Ausubel et al., 1989, *supra*), yet still encodes a functionally equivalent NHP product. Functional equivalents of a NHP include naturally occurring NHPs present in other species and mutant NHPs whether naturally occurring or engineered (by site directed mutagenesis, gene shuffling, directed evolution as described in, for example, U.S. Patents Nos. 5,837,458 and 5,723,323 both of which are herein incorporated by reference in their entirety). The invention also includes degenerate nucleic acid variants of the disclosed NHP polynucleotide sequences.

Additionally contemplated are polynucleotides encoding NHP ORFs, or their functional equivalents, encoded by polynucleotide sequences that are about 99, 95, 90, or about 85 percent similar or identical to corresponding regions of the nucleotide sequences of the Sequence Listing (as measured by BLAST sequence comparison analysis using, for example, the GCG sequence analysis package (Madison, Wisconsin) using standard default settings).

The invention also includes nucleic acid molecules, preferably DNA molecules, that hybridize to, and are therefore the complements of, the described NHP nucleotide sequences. Such hybridization conditions may be highly stringent or less highly stringent, as described above. In instances where the nucleic acid molecules are deoxyoligonucleotides ("DNA oligos"), such molecules are generally about 16 to about 100 bases long, or about 20 to about 80, or about 34 to about 45 bases long, or any variation or combination of sizes represented therein that incorporate a contiguous region of sequence first disclosed in the Sequence Listing. Such oligonucleotides can be used in conjunction with the polymerase chain reaction (PCR) to screen libraries, isolate clones, and prepare cloning and sequencing templates, etc.

Alternatively, such NHP oligonucleotides can be used as hybridization probes for screening libraries, and assessing gene expression patterns (particularly using a micro array or high-throughput "chip" format). Additionally, a series of the described NHP oligonucleotide sequences, or the complements thereof, can be used to represent all or a portion of the described NHP sequences. An oligonucleotide or polynucleotide sequence first disclosed in at least a portion of one or more of the sequences of SEQ ID NOS: 1-27 can be used as a hybridization probe in conjunction with a solid support matrix/substrate (resins, beads, membranes, plastics, polymers, metal or metallized substrates, crystalline or polycrystalline substrates, etc.). Of particular note are spatially addressable arrays (*i.e*., gene chips, microtiter plates, etc.) of oligonucleotides and polynucleotides, or corresponding oligopeptides and polypeptides, wherein at least one of the biopolymers present on the spatially addressable array comprises an oligonucleotide or polynucleotide sequence first disclosed in at least one of the sequences of SEQ ID NOS: 1-27, or an amino acid sequence encoded thereby. Methods for attaching biopolymers to, or synthesizing biopolymers on, solid support matrices, and conducting binding studies thereon are disclosed in, *inter alia*, U.S. Patent Nos. 5,700,637, 5,556,752, 5,744,305, 4,631,211, 5,445,934, 5,252,743, 4,713,326, 5,424,186, and 4,689,405 the disclosures of which are herein incorporated by reference in their entirety.

Addressable arrays comprising sequences first disclosed in SEQ ID NOS:1-27 can be used to identify and characterize the temporal and tissue specific expression of a gene. These addressable arrays incorporate oligonucleotide sequences of sufficient length to confer the required specificity, yet be within the limitations of the production technology. The length of these probes is within a range of between about 8 to about 2000 nucleotides. Preferably the probes consist of 60 nucleotides and more preferably 25 nucleotides from the sequences first disclosed in SEQ ID NOS:1-27.

For example, a series of the described oligonucleotide sequences, or the complements thereof, can be used in chip format to represent all or a portion of the described sequences. The oligonucleotides, typically between about 16 to about 40 (or any whole number within the stated range) nucleotides in length can partially overlap each other and/or the sequence may be represented using oligonucleotides that do not overlap. Accordingly, the described polynucleotide sequences shall typically comprise at least about two or three distinct oligonucleotide sequences of at least about 8 nucleotides in length that are each first disclosed in the described Sequence Listing. Such oligonucleotide sequences can begin at any nucleotide present within a sequence in the Sequence Listing and proceed in either a sense (5'-to-3') orientation vis-a-vis the described sequence or in an antisense orientation.

Microarray-based analysis allows the discovery of broad patterns of genetic activity, providing new understanding of gene functions and generating novel and unexpected insight into transcriptional processes and biological mechanisms. The use of addressable arrays comprising sequences first disclosed in SEQ ID NOS:1-27 provides detailed information about transcriptional changes involved in a specific pathway, potentially leading to the identification of novel components or gene functions that manifest themselves as novel phenotypes.

Probes consisting of sequences first disclosed in SEQ ID NOS:1-27 can also be used in the identification, selection and validation of novel molecular targets for drug discovery. The use of these unique sequences permits the direct confirmation of drug targets and recognition of drug dependent changes in gene expression that are modulated through pathways distinct from the drugs intended target. These unique sequences therefore also have utility in defining and monitoring both drug action and toxicity.

As an example of utility, the sequences first disclosed in SEQ ID NOS:1-27 can be utilized in microarrays or other assay formats, to screen collections of genetic material from patients who have a particular medical condition. These investigations can also be carried out using the sequences first disclosed in SEQ ID NOS:1-27 *in silico* and by comparing previously collected genetic databases and the disclosed sequences using computer software known to those in the art.

Thus the sequences first disclosed in SEQ ID NOS:1-27 can be used to identify mutations associated with a particular disease and also as a diagnostic or prognostic assay.

Although the presently described sequences have been specifically described using nucleotide sequence, it should be appreciated that each of the sequences can uniquely be described using any of a wide variety of additional structural attributes, or combinations thereof. For example, a given sequence can be described by the net composition of the nucleotides present within a given region of the sequence in conjunction with the presence of one or more specific oligonucleotide sequence(s) first disclosed in the SEQ ID NOS: 1-27. Alternatively, a restriction map specifying the relative positions of restriction endonuclease digestion sites, or various palindromic or other specific oligonucleotide sequences can be used to structurally describe a given sequence. Such restriction maps, which are typically generated by widely available computer programs (*e.g*., the University of Wisconsin GCG sequence analysis package, SEQUENCHER 3.0, Gene Codes Corp., Ann Arbor, MI, etc.), can optionally be used in conjunction with one or more discrete nucleotide sequence(s) present in the sequence that can be described by the relative position of the sequence relatve to one or more additional sequence(s) or one or more restriction sites present in the disclosed sequence.

For oligonucleotide probes, highly stringent conditions may refer, *e*.*g*., to washing in 6xSSC/0.05% sodium pyrophosphate at 37°C (for 14-base oligos), 48°C (for 17-base oligos), 55°C (for 20-base oligos), and 60°C (for 23-base oligos). These nucleic acid molecules may encode or act as NHP gene antisense molecules, useful, for example, in NHP gene regulation (for and/or as antisense primers in amplification reactions of NHP nucleic acid sequences). With respect to NHP gene regulation, such techniques can be used to regulate biological functions. Further, such sequences may be used as part of ribozyme and/or triple helix sequences that are also useful for NHP gene regulation.

Inhibitory antisense or double stranded oligonucleotides can additionally comprise at least one modified base moiety which is selected from the group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar moiety selected from the group including but not limited to arabinose, 2-fluoroarabinose, xylulose, and hexose.

In yet another embodiment, the antisense oligonucleotide will comprise at least one modified phosphate backbone selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. 15:6625-6641). The oligonucleotide is a 2'-0-methylribonucleotide (Inoue *et al*., 1987, Nucl. Acids Res. *15*:6131-6148), or a chimeric RNA-DNA analogue (Inoue *et al*., 1987, FEBS Lett. *215*:327-330). Alternatively, double stranded RNA can be used to disrupt the expression and function of a targeted NHP.

Oligonucleotides of the invention can be synthesized by standard methods known in the art, *e.g*. by use of an automated DNA synthesizer (such as are commercially available from Biosearch, Applied Biosystems, etc.). As examples, phosphorothioate oligonucleotides can be synthesized by the method of Stein *et al*. (1988, Nucl. Acids Res. 16:3209), and methylphosphonate oligonucleotides can be prepared by use of controlled pore glass polymer supports (Sarin *et al*., 1988, Proc. Natl. Acad. Sci. U.S.A. *85*:7448-7451), etc.

Low stringency conditions are well known to those of skill in the art, and will vary predictably depending on the specific organisms from which the library and the labeled sequences are derived. For guidance regarding such conditions see, for example, Sambrook *et al*., 1989, Molecular Cloning, A Laboratory Manual (and periodic updates thereof), Cold Springs Harbor Press, N.Y.; and Ausubel *et al.,* 1989, Current Protocols in Molecular Biology, Green Publishing Associates and Wiley Interscience, N.Y.

Alternatively, suitably labeled NHP nucleotide probes can be used to screen a human genomic library using appropriately stringent conditions or by PCR. The identification and characterization of human genomic clones is helpful for identifying polymorphisms (including, but not limited to, nucleotide repeats, microsatellite alleles, single nucleotide polymorphisms, or coding single nucleotide polymorphisms), determining the genomic structure of a given locus/allele, and designing diagnostic tests. For example, sequences derived from regions adjacent to the intron/exon boundaries of the human gene can be used to design primers for use in amplification assays to detect mutations within the exons, introns, splice sites (e.g., splice acceptor and/or donor sites), etc., that can be used in diagnostics and pharmacogenomics.

Further, a NHP gene homolog can be isolated from nucleic acid from an organism of interest by performing PCR using two degenerate or "wobble" oligonucleotide primer pools designed on the basis of amino acid sequences within the NHP products disclosed herein. The template for the reaction may be total RNA, mRNA, and/or cDNA obtained by reverse transcription of mRNA prepared from human or non-human cell lines or tissue known or suspected to express an allele of a NHP gene.

The PCR product can be subcloned and sequenced to ensure that the amplified sequences represent the sequence of the desired NHP gene. The PCR fragment can then be used to isolate a full length cDNA clone by a variety of methods. For example, the amplified fragment can be labeled and used to screen a cDNA library, such as a bacteriophage cDNA library. Alternatively, the labeled fragment can be used to isolate genomic clones via the screening of a genomic library.

PCR technology can also be used to isolate full length cDNA sequences. For example, RNA can be isolated, following standard procedures, from an appropriate cellular or tissue source (*i*.*e*., one known, or suspected, to express a NHP sequence). A reverse transcription (RT) reaction can be performed on the RNA using an oligonucleotide primer specific for the most 5' end of the amplified fragment for the priming of first strand synthesis. The resulting RNA/DNA hybrid may then be "tailed" using a standard terminal transferase reaction, the hybrid may be digested with RNase H, and second strand synthesis may then be primed with a complementary primer. Thus, cDNA sequences upstream of the amplified fragment can be isolated. For a review of cloning strategies that can be used, see *e*.*g*., Sambrook *et al*., 1989, *supra.*

A cDNA encoding a mutant NHP sequence can be isolated, for example, by using PCR. In this case, the first cDNA strand may be synthesized by hybridizing an oligo-dT oligonucleotide to mRNA isolated from tissue known or suspected to be expressed in an individual putatively carrying a mutant NHP allele, and by extending the new strand with reverse transcriptase. The second strand of the cDNA is then synthesized using an oligonucleotide that hybridizes specifically to the 5' end of the normal gene. Using these two primers, the product is then amplified via PCR, optionally cloned into a suitable vector, and subjected to DNA sequence analysis through methods well known to those of skill in the art. By comparing the DNA sequence of the mutant NHP allele to that of a corresponding normal NHP allele, the mutation(s) responsible for the loss or alteration of function of the mutant NHP gene product can be ascertained.

Alternatively, a genomic library can be constructed using DNA obtained from an individual suspected of or known to carry a mutant NHP allele (*e.g*., a person manifesting a NHP-associated phenotype such as, for example, obesity, vision disorders, high blood pressure, depression, seizures, infertility, etc.), or a cDNA library can be constructed using RNA from a tissue known, or suspected, to express a mutant NHP allele. A normal NHP gene, or any suitable fragment thereof, can then be labeled and used as a probe to identify the corresponding mutant NHP allele in such libraries. Clones containing mutant NHP gene sequences can then be purified and subjected to sequence analysis according to methods well known to those skilled in the art.

Additionally, an expression library can be constructed utilizing cDNA synthesized from, for example, RNA isolated from a tissue known, or suspected, to express a mutant NHP allele in an individual suspected of or known to carry such a mutant allele. In this manner, gene products made by the putatively mutant tissue can be expressed and screened using standard antibody screening techniques in conjunction with antibodies raised against a normal NHP product, as described below. (For screening techniques, see, for example, Harlow, E. and Lane, eds., 1988, "Antibodies: A Laboratory Manual", Cold Spring Harbor Press, Cold Spring Harbor.)

Additionally, screening can be accomplished by screening with labeled NHP fusion proteins, such as, for example, alkaline phosphatase-NHP or NHP-alkaline phosphatase fusion proteins. In cases where a NHP mutation results in an expressed gene product with altered function (*e.g*., as a result of a missense or a frameshift mutation), polyclonal antibodies to a NHP are likely to cross-react with a corresponding mutant NHP gene product. Library clones detected via their reaction with such labeled antibodies can be purified and subjected to sequence analysis according to methods well known in the art.

The invention also encompasses (a) DNA vectors that contain any of the foregoing NHP coding sequences and/or their complements (*i.e*., antisense); (b) DNA expression vectors that contain any of the foregoing NHP coding sequences operatively associated with a regulatory element that directs the expression of the coding sequences (for example, baculo virus as described in U.S. Patent No. 5,869,336 herein incorporated by reference); (c) genetically engineered host cells that contain any of the foregoing NHP coding sequences operatively associated with a regulatory element that directs the expression of the coding sequences in the host cell; and (d) genetically engineered host cells that express an endogenous NHP gene under the control of an exogenously introduced regulatory element (*i.e*., gene activation). As used herein, regulatory elements include, but are not limited to, inducible and non-inducible promoters, enhancers, operators and other elements known to those skilled in the art that drive and regulate expression. Such regulatory elements include but are not limited to the cytomegalovirus (hCMV) immediate early gene, regulatable, viral elements (particularly retroviral LTR promoters), the early or late promoters of SV40 adenovirus, the *lac* system, the *trp* system, the *TAC* system, the TRC system, the major operator and promoter regions of phage lambda, the control regions of fd coat protein, the promoter for 3-phosphoglycerate kinase (PGK), the promoters of acid phosphatase, and the promoters of the yeast α-mating factors.

The present invention also encompasses antibodies and anti-idiotypic antibodies (including Fab fragments), antagonists and agonists of the NHP, as well as compounds or nucleotide constructs that inhibit expression of a NHP gene (transcription factor inhibitors, antisense and ribozyme molecules, or gene or regulatory sequence replacement constructs), or promote the expression of a NHP (*e*.*g*., expression constructs in which NHP coding sequences are operatively associated with expression control elements such as promoters, promoter/enhancers, etc.).

The NHPs or NHP peptides, NHP fusion proteins, NHP nucleotide sequences, antibodies, antagonists and agonists can be useful for the detection of mutant NHPs or inappropriately expressed NHPs for the diagnosis of disease. The NHP proteins or peptides, NHP fusion proteins, NHP nucleotide sequences, host cell expression systems, antibodies, antagonists, agonists and genetically engineered cells and non-human animals can be used for screening for drugs (or high throughput screening of combinatorial libraries) effective in the treatment of the symptomatic or phenotypic manifestations of perturbing the normal function of NHP in the body. The use of engineered host cells and/or non-human animals may offer an advantage in that such systems allow not only for the identification of compounds that bind to the endogenous receptor for an NHP, but can also identify compounds that trigger NHP-mediated activities or pathways.

Finally, the NHP products can be used as therapeutics. For example, soluble derivatives such as NHP peptides/domains corresponding to the NHPs, secreted forms of a NHP, NHP fusion protein products (especially NHP-Ig fusion proteins, *i*.*e*., fusions of a NHP, or a domain of a NHP, to an IgFc), NHP antibodies and anti-idiotypic antibodies (including Fab fragments), antagonists or agonists (including compounds that modulate or act on downstream targets in a NHP-mediated pathway) can be used to directly treat diseases or disorders. For instance, the administration of an effective amount of soluble NHP, or a NHP-IgFc fusion protein or an anti-idiotypic antibody (or its Fab) that mimics the NHP could activate or effectively antagonize the endogenous NHP receptor. Nucleotide constructs encoding such NHP products can be used to genetically engineer host cells to express such products *in vivo*; these genetically engineered cells function as "bioreactors" in the body delivering a continuous supply of a NHP, a NHP peptide, or a NHP fusion protein to the body. Nucleotide constructs encoding functional NHPs, mutant NHPs, as well as antisense and ribozyme molecules can also be used in "gene therapy" approaches for the modulation of NHP expression. Thus, the invention also encompasses pharmaceutical formulations and methods for treating biological disorders.

Various aspects of the invention are described in greater detail in the subsections below.

### 5.1 THE NHP SEQUENCES

The cDNA sequences and the corresponding deduced amino acid sequences of the described NHPs are presented in the Sequence Listing. The NHP nucleotides were obtained from clustered human gene trapped sequences, ESTs, and cDNAs isolated from human brain, fetal brain, cerebellum, and hypothalamus cDNA libraries (Edge Biosystems, Gaithersburg, MD). The described sequences share limited structural similarity with a variety of proteins, including, but not limited to, neurexins (including secreted types) and contactin associated proteins. A polymorphism was identified that results in a C-or-T transition at, for example, the position corresponding to nucleotide 812 of SEQ ID NO:1 that can result in a ser or leu being present at, for example, amino acid position 271 of SEQ ID NO:2.

### 5.2 NHPS AND NHP POLYPEPTIDES

NHPs, polypeptides, peptide fragments, mutated, truncated, or deleted forms of the NHPs, and/or NHP fusion proteins can be prepared for a variety of uses. These uses include, but are not limited to, the generation of antibodies, as reagents in diagnostic assays, for the identification of other cellular gene products related to a NHP, as reagents in assays for screening for compounds that can be as pharmaceutical reagents useful in the therapeutic treatment of mental, biological, or medical disorders and disease. Given the similarity information and expression data, the described NHPs can be targeted (by drugs, oligos, antibodies, etc,) in order to treat disease, or to therapeutically augment the efficacy of therapeutic agents.

The Sequence Listing discloses the amino acid sequences encoded by the described NHP sequences. The NHPs typically display initiator methionines in DNA sequence contexts consistent with a translation initiation site, and signal sequences characteristic of membrane or secreted proteins.

The NHP amino acid sequences of the invention include the amino acid sequences presented in the Sequence Listing as well as analogues and derivatives thereof. Further, corresponding NHP homologues from other species are encompassed by the invention. In fact, any NHP protein encoded by the NHP nucleotide sequences described above are within the scope of the invention, as are any novel polynucleotide sequences encoding all or any novel portion of an amino acid sequence presented in the Sequence Listing. The degenerate nature of the genetic code is well known, and, accordingly, each amino acid presented in the Sequence Listing, is generically representative of the well known nucleic acid "triplet" codon, or in many cases codons, that can encode the amino acid. As such, as contemplated herein, the amino acid sequences presented in the Sequence Listing, when taken together with the genetic code (see, for example, Table 4-1 at page 109 of "Molecular Cell Biology", 1986, J. Darnell *et al*. eds., Scientific American Books, New York, NY, herein incorporated by reference) are generically representative of all the various permutations and combinations of nucleic acid sequences that can encode such amino acid sequences.

The invention also encompasses proteins that are functionally equivalent to the NHPs encoded by the presently described nucleotide sequences as judged by any of a number of criteria, including, but not limited to, the ability to bind and cleave a substrate of a NHP, or the ability to effect an identical or complementary downstream pathway, or a change in cellular metabolism (*e*.*g*., proteolytic activity, ion flux, tyrosine phosphorylation, transport, etc.). Such functionally equivalent NHP proteins include, but are not limited to, additions or substitutions of amino acid residues within the amino acid sequence encoded by the NHP nucleotide sequences described above, but which result in a silent change, thus producing a functionally equivalent gene product. Amino acid substitutions may be made on the basis of similarity in polarity, charge, solubility, hydrophobicity, hydrophilicity, and/or the amphipathic nature of the residues involved. For example, nonpolar (hydrophobic) amino acids include alanine, leucine, isoleucine, valine, proline, phenylalanine, tryptophan, and methionine; polar neutral amino acids include glycine, serine, threonine, cysteine, tyrosine, asparagine, and glutamine; positively charged (basic) amino acids include arginine, lysine, and histidine; and negatively charged (acidic) amino acids include aspartic acid and glutamic acid.

A variety of host-expression vector systems can be used to express the NHP nucleotide sequences of the invention. Where, as in the present instance, the NHP peptides or polypeptides are thought to be membrane proteins, the hydrophobic regions of the protein can be excised at the protein or nucleic acid level (*i.e*., the hydrophobic region spanning from roughly about amino acid number 1,240 to about amino acid position 1,265 of, for example, SEQ ID NO:2) and the resulting soluble peptide or polypeptide can be recovered from the culture media. Such expression systems also encompass engineered host cells that express a NHP, or a functional equivalent, *in situ*. Purification or enrichment of a NHP from such expression systems can be accomplished using appropriate detergents and lipid micelles and methods well known to those skilled in the art. However, such engineered host cells themselves may be used in situations where it is important not only to retain the structural and functional characteristics of the NHP, but to assess biological activity, *e*.*g*., in drug screening assays.

The expression systems that can be used for purposes of the invention include but are not limited to microorganisms such as bacteria (*e*.*g*., *E. coli, B. subtilis*) transformed with recombinant bacteriophage DNA, plasmid DNA or cosmid DNA expression vectors containing NHP nucleotide sequences; yeast (*e*.*g*., *Saccharomyces*, *Pichia*) transformed with recombinant yeast expression vectors containing NHP nucleotide sequences; insect cell systems infected with recombinant virus expression vectors (*e*.*g*., baculovirus) containing NHP sequences; plant cell systems infected with recombinant virus expression vectors (*e.g*., cauliflower mosaic virus, CaMV; tobacco mosaic virus, TMV) or transformed with recombinant plasmid expression vectors (*e*.*g*., Ti plasmid) containing NHP nucleotide sequences; or mammalian cell systems (*e*.*g*., COS, CHO, BHK, 293, 3T3) harboring recombinant expression constructs containing promoters derived from the genome of mammalian cells (*e.g*., metallothionein promoter) or from mammalian viruses (*e.g*., the adenovirus late promoter; the vaccinia virus 7.5K promoter).

In bacterial systems, a number of expression vectors may be advantageously selected depending upon the use intended for the NHP product being expressed. For example, when a large quantity of such a protein is to be produced for the generation of pharmaceutical compositions of or containing NHP, or for raising antibodies to a NHP, vectors that direct the expression of high levels of fusion protein products that are readily purified may be desirable. Such vectors include, but are not limited, to the *E*. *coli* expression vector pUR278 (Ruther *et al*., 1983, EMBO J. 2:1791), in which a NHP coding sequence may be ligated individually into the vector in frame with the *lacZ* coding region so that a fusion protein is produced; pIN vectors (Inouye & Inouye, 1985, Nucleic Acids Res. 13:3101-3109; Van Heeke & Schuster, 1989, J. Biol. Chem. 264:5503-5509); and the like. pGEX vectors (Pharmacia or American Type Culture Collection) can also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. The PGEX vectors are designed to include thrombin or factor Xa protease cleavage sites so that the cloned target gene product can be released from the GST moiety.

In an insect system, *Autographa californica* nuclear polyhidrosis virus (AcNPV) is used as a vector to express foreign genes. The virus grows in *Spodoptera frugiperda* cells. A NHP coding sequence may be cloned individually into non-essential regions (for example the polyhedrin gene) of the virus and placed under control of an AcNPV promoter (for example the polyhedrin promoter). Successful insertion of NHP coding sequence will result in inactivation of the polyhedrin gene and production of non-occluded recombinant virus (*i.e*., virus lacking the proteinaceous coat coded for by the polyhedrin gene). These recombinant viruses are then used to infect Spodoptera frugiperda cells in which the inserted sequence is expressed (*e*.*g*., see Smith *et al*., 1983, J. Virol. *46*:584; Smith, U.S. Patent No. 4,215,051).

In mammalian host cells, a number of viral-based expression systems may be utilized. In cases where an adenovirus is used as an expression vector, the NHP nucleotide sequence of interest may be ligated to an adenovirus transcription/translation control complex, *e.g*., the late promoter and tripartite leader sequence. This chimeric gene may then be inserted in the adenovirus genome by *in vitro* or *in vivo* recombination. Insertion in a non-essential region of the viral genome (*e.g*., region E1 or E3) will result in a recombinant virus that is viable and capable of expressing a NHP product in infected hosts (*e.g*., See Logan & Shenk, 1984, Proc. Natl. Acad. Sci. USA 81:3655-3659). Specific initiation signals may also be required for efficient translation of inserted NHP nucleotide sequences. These signals include the ATG initiation codon and adjacent sequences. In cases where an entire NHP gene or cDNA, including its own initiation codon and adjacent sequences, is inserted into the appropriate expression vector, no additional translational control signals may be needed. However, in cases where only a portion of a NHP coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (See Bitter *et al*., 1987, Methods in Enzymol. *153*:516-544).

In addition, a host cell strain may be chosen that modulates the expression of the inserted sequences, or modifies and processes the gene product in the specific fashion desired. Such modifications (*e.g*., glycosylation) and processing (*e.g*., cleavage) of protein products may be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product may be used. Such mammalian host cells include, but are not limited to, CHO, VERO, BHK, HeLa, COS, MDCK, 293, 3T3, WI38, and in particular, human cell lines.

For long-term, high-yield production of recombinant proteins, stable expression is preferred. For example, cell lines which stably express the NHP sequences described above can be engineered. Rather than using expression vectors which contain viral origins of replication, host cells can be transformed with DNA controlled by appropriate expression control elements (*e*.*g*., promoter, enhancer sequences, transcription terminators, polyadenylation sites, etc.), and a selectable marker. Following the introduction of the foreign DNA, engineered cells may be allowed to grow for 1-2 days in an enriched media, and then are switched to a selective media. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the NHP product. Such engineered cell lines may be particularly useful in screening and evaluation of compounds that affect the endogenous activity of the NHP product.

A number of selection systems may be used, including but not limited to the herpes simplex virus thymidine kinase (Wigler, *et al*., 1977, Cell 11:223), hypoxanthine-guanine phosphoribosyltransferase (Szybalska & Szybalski, 1962, Proc. Natl. Acad. Sci. USA *48*:2026), and adenine phosphoribosyltransferase (Lowy, *et al*., 1980, Cell *22*:817) genes can be employed in tk⁻, hgprt⁻ or aprt⁻ cells, respectively. Also, antimetabolite resistance can be used as the basis of selection for the following genes: dhfr, which confers resistance to methotrexate (Wigler, *et al*., 1980, Natl. Acad. Sci. USA 77:3567; O'Hare, *et al*., 1981, Proc. Natl. Acad. Sci. USA *78*:1527); gpt, which confers resistance to mycophenolic acid (Mulligan & Berg, 1981, Proc. Natl. Acad. Sci. USA *78*:2072); neo, which confers resistance to the aminoglycoside G-418 (Colberre-Garapin, *et al*., 1981, J. Mol. Biol. 150:1); and hygro, which confers resistance to hygromycin (Santerre, *et al*., 1984, Gene *30*:147).

Alternatively, any fusion protein can be readily purified by utilizing an antibody specific for the fusion protein being expressed. For example, a system described by Janknecht *et al*. allows for the ready purification of non-denatured fusion proteins expressed in human cell lines (Janknecht, *et al*., 1991, Proc. Natl. Acad. Sci. USA *88*:8972-8976). In this system, the gene of interest is subcloned into a vaccinia recombination plasmid such that the gene's open reading frame is translationally fused to an amino-terminal tag consisting of six histidine residues. Extracts from cells infected with recombinant vaccinia virus are loaded onto Ni²⁺·nitriloacetic acid-agarose columns and histidine-tagged proteins are selectively eluted with imidazole-containing buffers.

Also encompassed by the present invention are fusion proteins that direct the NHP to a target organ and/or facilitate transport across the membrane into the cytosol. Conjugation of NHPs to antibody molecules or their Fab fragments could be used to target cells bearing a particular epitope. Attaching the appropriate signal sequence to the NHP would also transport the NHP to the desired location within the cell. Alternatively targeting of NHP or its nucleic acid sequence might be achieved using liposome or lipid complex based delivery systems. Such technologies are described in Liposomes:A Practical Approach, New,RRC ed., Oxford University Press, New York and in U.S. Patents Nos. 4,594,595, 5,459,127, 5,948,767 and 6,110,490 and their respective disclosures which are herein incorporated by reference in their entirety. Additionally embodied are novel protein constructs engineered in such a way that they facilitate transport of the NHP to the target site or desired organ, where they cross the cell membrane and/or the nucleus where the NHP can exert its functional activity. This goal may be achieved by coupling of the NHP to a cytokine or other ligand that provides targeting specificity, and/or to a protein transducing domain (see generally U.S. applications Ser. No. 60/111, 701 and 60/056, 713, both of which are herein incorporated by reference, for examples of such transducing sequences) to facilitate passage across cellular membranes and can optionally be engineered to include nuclear localization sequences.

## Claims

1. An isolated nucleic acid molecule comprising the nucleotide sequence first disclosed in SEQ ID NO: 1.

2. An isolated nucleic acid molecule comprising a nucleotide sequence that:
(a) encodes the amino acid sequence shown in SEQ ID NO: 2: and
(b) hybridizes under stringent conditions to the nucleotide sequence of SEQ ID NO: 1 or the complement thereof.

3. An isolated nucleic acid molecule comprising a nucleotide sequence that encodes the amino acid sequence shown in SEQ ID NO: 2.

4. An isolated nucleic acid molecule comprising a nucleotide sequence that encodes the amino acid sequence shown in SEQ ID NO: 4.

5. An isolated nucleic acid molecule comprising a nucleotide sequence that encodes the amino acid sequence shown in SEQ ID NO: 24.

6. A polypeptide encoded by a nucleic acid as claimed in any one of claims 1 to 5.

7. A host cell that comprises a nucleic acid as claimed in any one of claims 1 to 5 and is capable of expressing the polypeptide encoded by said nucleic acid, optionally in a soluble form.

8. A non-human animal that is capable of expressing a nucleic acid as claimed in any one of claims 1 to 5, or that is an optionally conditional "knock-out" animal that does not express a polypeptide as claimed in claim 6.

9. Use of a nucleic acid as claimed in any one of claims 1 to 5, a polypeptide as claimed in claim 6, a host cell as claimed in claim 7 or an animal as claimed in claim 8, in a method for screening compounds for the ability to modulate the expression of a nucleic acid as claimed in any one of claims 1 to 5 or a polypeptide as claimed in claim 6, or that modulates the activity of a polypeptide as claimed claim 6, for example, for high throughput screening of combinatorial libraries.

## Revendications

1. Une molécule d'acide nucléique isolée comprenant la séquence de nucléotides décrite en premier dans la SEQ ID No 1.

2. Une molécule d'acide nucléique isolée comprenant une séquence de nucléotides qui :
(a) code pour la séquence d'acides aminés montrée dans la SEQ ID No : 2 ; et
(b) s'hybride dans des conditions rigoureuses à la séquence de nucléotides de la SEQ. ID No 1 ou à son complément.

3. Une molécule d'acide nucléique isolée comprenant une séquence de nucléotides qui code pour la séquence d'acides aminés montrée dans la SEQ. ID No : 2.

4. Une molécule d'acide nucléique isolée comprenant une séquence de nucléotides qui code pour la séquence d'acides aminés montrée dans la SEQ. ID No : 4.

5. Une molécule d'acide nucléique isolée comprenant une séquence de nucléotides qui code pour la séquence d'acides aminés montrée dans la SEQ. ID No : 24.

6. Un polypeptide codé par un acide nucléique selon l'une quelconque des revendications 1 à 5.

7. Une cellule hôte qui comprend un acide nucléique selon l'une quelconque des revendications 1 à 5 et est capable d'exprimer le polypeptide codé par le dit acide nucléique, éventuellement dans une forme soluble.

8. Un animal non humain qui est capable d'exprimer un acide nucléique selon l'une quelconque des revendications 1 à 5, ou qui est un animal " knock-out " éventuellement conditionnel qui n'exprime pas un polypeptide selon la revendication 6.

9. Utilisation d'un acide nucléique selon l'une quelconque des revendications 1 à 5, d'un polypeptide selon la revendication 6, d'une cellule hôte selon la revendication 7 ou d'un animal selon la revendication 8, dans une méthode de criblage de composés pour déterminer l'aptitude à moduler l'expression d'un acide nucléique selon l'une quelconque des revendications 1 à 5 ou d'un polypeptide selon la revendication 6, ou qui module l'activité d'un polypeptide selon la revendication 6, par exemple, pour un criblage à haut débit de librairies combinatoires.

## Patentansprüche

1. Ein isoliertes Nukleinsäuremolekül umfassend die Nukleotidsequenz zuerst offenbart in SEQ ID Nr: 1.

2. Ein isoliertes Nukleinsäuremolekül umfassend eine Nukleotidsequenz, welche:
(a) Für die in SEQ ID Nr: 2 gezeigte Aminosäuresequenz codiert; und
(b) unter stringenten Bedingungen mit der Nukleotidsequenz aus SEQ ID Nr: 1 oder der Komplementärsequenz davon hybridisiert.

3. Ein isoliertes Nukleinsäuremolekül umfassend eine Nukleotidsequenz, welche für die in SEQ ID Nr: 2 gezeigte Aminosäuresequenz codiert.

4. Ein isoliertes Nukleinsäuremolekül umfassend eine Nukleotidsequenz, welche für die in SEQ ID Nr: 4 gezeigte Aminosäuresequenz codiert.

5. Ein isoliertes Nukleinsäuremolekül aufweisend eine Nukleotidsequenz, welche für die in SEQ ID Nr: 24 gezeigte Aminosäuresequenz codiert.

6. Ein Polypeptid codiert durch eine Nukleinsäure wie in einem der Ansprüche 1 bis 5 beansprucht.

7. Eine Wirtszelle, die eine Nukleinsäure umfasst wie in einem der Ansprüche 1 bis 5 beansprucht und die in der Lage ist, das Polypeptid, das durch die Nukleinsäure codiert wird, gegebenenfalls in einer löslichen Form zu exprimieren.

8. Ein nicht-menschliches Tier, das in der Lage ist, eine Nukleinsäure wie in einem der Ansprüche 1 bis 5 beansprucht, zu exprimieren, oder welches ein optional konditionales "knock-out"-Tier ist, das nicht ein Polypeptid wie in Anspruch 6 beansprucht exprimiert.

9. Verwendung einer Nukleinsäure wie in einem der Ansprüche 1 bis 5 beansprucht, eines Polypeptids wie in Anspruch 6 beansprucht, einer Wirtszelle wie in Anspruch 7 beansprucht oder eines Tieres wie in Anspruch 8 beansprucht, in einem Verfahren zum Screening von Verbindungen auf die Fähigkeit die Expression einer Nukleinsäure wie in einem der Ansprüche 1 bis 5 beansprucht oder eines Polypeptids wie in Anspruch 6 beansprucht zu modulieren, oder welche die Aktivität eines Polypeptids wie in Anspruch 6 beansprucht modulieren, beispielsweise zum Hochdurchsatz-Screening von kombinatorischen Bibliotheken.


### SEQUENCE LISTING

<110> LEXICON GENETICS INCORPORATED
<120> Novel Human Neurexin-like Proteins and Polynucleotides Encoding the Same
<130> LEX-0122-PCT
<150> 00 00 176, 557
   <151> 2000-61-26
<152> US 00 199, 513
   <191> 2000-04-25
<160> 27
<170> FastSEQ for Windows Version 4.0
<210> 1
   <211> 3924
   <212> DNA
   <213> homo sapiens
<400> 1
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaagctat tgcactcagt gagagcccga tttgttcgct ttgtgcccct ggaatggaat     480
   cccagtggga agattggcat gagagtcgag gtctacggat gttcctataa atcagacgtt     540
   gctgactttg atggccgaag ctcacttctg tacaggttca atcagaagtt gatgagtact     600
   ctcaaagatg tgatctccct gaagttcaag agcatgcaag gagatggggt cctgttccat     660
   ggagaaggtc agcgtggaga ccacatcacc ttggaactcc agaaggggag gctcgcccta     720
   cacctcaatt tgggtgacag caaagcgcgg ctcagcagca gcttgccctc tgccaccctg     780
   ggcagcctcc tggatgacca gcactggcac tyggtcctca ttgagcgggt gggcaagcag     840
   gtgaacttca cggtggacaa gcacacacag cacttccgca ccaagggcga gacggatgcc     900
   ttagacattg actatgagct tagttttgga ggaattccag taccaggaaa acctgggacc     960
   tttttaaaga aaaacttcca tggatgcatc gaaaaccttt actacaatgg agtaaacata    1020
   attracctgg ctaagagacg aaagcatcag atctatactg tgggcaatgt cactttttcc    1080
   tgctccgaac cacagattgt gcccatcaca tttgtyaact ccagcggcag ctatttgctg    1140
   ctgcccggca ccccccaaat tgatgggctc tcagtgagtt tccagtttcg aacatggaac    1200
   aaggatggtc tgcttctgtc cacagagctg tctgagggct cgggaaccct gctgctgagc    1260
   ctggagggtg gaatcctgag actcgtgatt cagaaaatga cagaacgcgt agctgaaatc    1320
   ctcacaggca gcaacttgaa tgatggcctg tggcactcgg ttagcatcaa cgccaggagg    1380
   aaccgcatca cgctcactct ggatgatgaa gcagcacccc cggctccaga cagcacttgg    1440
   gtgcagattt attctggaaa tagctactat tttggagggt gccccgacaa tctcaccgat    1500
   tcccaatgtt taaatcccat taaggctttc caaggctgca tgaggctcat ctttattgat    1560
   aaccagccca aggacctcat ttcagttcag caaggttccc tggggaattt tagtgattta    1620
   cacattgatc tgtgtagcat caaagacagg tgtttgccaa actactgtga acatggagga    1680
   agctgctccc agtcctggac taccttctat tgtaactgca gtgacacaag ttacactggt    1740
   gccacctgcc acaactccat ctacgagcaa tcctgcgagg tgtacaggca ccaggggaat    1800
   acagccggct tcttctacat cgactcagat ggcagcggcc cactgggacc tctccaggtg   1860
   tactgcaata tcactgagga caagatctgg acatcagtgc agcacaacaa tacagagctg   1920
   acccgagtgc ggggcgctaa ccctgagaag ccctatgcca tggccttgga ctacgggggc   1980
   agcatggaac agctggaggc cgtgatcgac ggctctgagc actgtgagca ggaggtggcc   2040
   taccactgca ggaggtcccg cctgctcaac acgccggatg gaacaccatt tacctggtgg   2100
   attgggcggt ccaatgaaag gcacccttac tggggaggtt cccctcctgg ggtccagcag   2160
   tgtgagtgtg gcctagacga gagctgcctg gacattcagc acttttgcaa ttgcgacgct   2220
   gacaaggatg aatggacaaa tgatactggc tttctttcct tcaaagacca cttgcctgtc   2280
   actcagatag ttatcactga taccgacaga tcaaactcag aagccgcttg gagaattggt   2340
   cccttgcgtt gctatggtga ccgacgcttc tggaacgccg tctcatttta tacagaagcc   2400
   tcttacctcc actttcctac cttccatgcg gaattcagtg ccgatatttc cttctttttt   2460
   aaaaccacag cattatccgg agttttccta gaaaatcttg gcattaaaga cttcattcga   2520
   ctcgaaataa gctctccttc agagatcacc tttgccatcg atgttgggaa tggtcctgtg   2580
   gagcttgtag tccagtctcc ttctcttctg aatgacaacc aatggcacta tgtccgggct   2640
   gagaggaacc tcaaggagac ctccctgcag gtggacaacc ttccaaggag caccagggag   2700
   acgtcggagg agggccattt tcgactgcag ctgaacagcc agttgtttgt agggggaacg   2760
   tcatccagac agaaaggctt cctaggatgc attcgctcct tacacttgaa tggacagaaa   2820
   atggacctgg aagagagggc aaaggtcaca tctggagtca ggccaggctg ccccggccac   2880
   tgcagcagct acggcagcat ctgccacaac gggggcaagt gtgtggagaa gcacaatggc   2940
   tacctgtgtg attgcaccaa ttcaccttat gaagggccct tttgcaaaaa agaggtttct   3000
   gctgtttttg aggctggcac gtcggttact tacatgtttc aagaacccta tcctgtgacc   3060
   aagaatataa gcctctcatc ctcagctatt tacacagatt cagctccatc caaggaaaac   3120
   attgcactta gctttgtgac aacccaggca cccagtcttt tgctctttat caattcttct   3180
   tctcaggact tcgtggttgt tctgctctgc aagaatggaa gcttacaggt tcgctatcac   3240
   ctaaacaagg aagaaaccca tgtattcacc attgatgcag ataactttgc taacagaagg   3300
   atgcaccact tgaagattaa ccgagaggga agagagctta ccattcagat ggaccagcaa   3360
   cttcgactca gttataactt ctctccggaa gtagagttca gggttataag gtcactcacc   3420
   ttgggcaaag tcacagagaa tcttggtttg gattctgaag ttgctaaagc aaatgccatg   3480
   ggttttgctg gatgcatgtc ttccgtccag tacaaccaca tagcaccact gaaggctgcc   3540
   ctgcgccatg ccactgtcgc gcctgtgact gtccatggga ccttgacgga atccagctgt   3600
   ggcttcatgg tggactcaga tgtgaatgca gtgaccacgg tgcattcttc atcagatcct   3660
   tttgggaaga cagatgagcg ggaaccactc acaaatgctg ttcgaagtga ttcggcagtc   3720
   atcggagggg tgatagcagt ggtgatattc atcatcttct gtatcatcgg catcatgacc   3780
   cggttcctct accagcacaa gcagtcacat cgtacgagcc agatgaagga gaaggaatat   3840
   ccagaaaatt tggacagttc cttcagaaat gaaattgact tgcaaaacac agtgagcgag   3900
   tgtaaacggg aatatttcat ctga                                          3924
<210> 2
   <211> 1307
   <212> PRT
   <213> homo sapiens
<220>
   <221> VARIANT
   <222> (1)...(1307)
   <223> Xaa = Any Amino Acid
<400> 2
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Leu Ser Phe Gly Gly Ile Pro Val Pro Gly Lys Pro Gly Thr
   305                 310                 315                 320
   Phe Leu Lys Lys Asn Phe His Gly Cys Ile Glu Asn Leu Tyr Tyr Asn
                   325                 330                 335
   Gly Val Asn Ile Ile Xaa Leu Ala Lys Arg Arg Lys His Gln Ile Tyr
               340                 345                 350
   Thr Val Gly Asn Val Thr Phe Ser Cys Ser Glu Pro Gln Ile Val Pro
           355                 360                 365
   Ile Thr Phe Val Asn Ser Ser Gly Ser Tyr Leu Leu Leu Pro Gly Thr
       370                 375                 380
   Pro Gln Ile Asp Gly Leu Ser Val Ser Phe Gln Phe Arg Thr Trp Asn
   385                 390                 395                 400
   Lys Asp Gly Leu Leu Leu Ser Thr Glu Leu Ser Glu Gly Ser Gly Thr
                   405                 410                 415
   Leu Leu Leu Ser Leu Glu Gly Gly Ile Leu Arg Leu Val Ile Gln Lys
               420                 425                 430
   Met Thr Glu Arg Val Ala Glu Ile Leu Thr Gly Ser Asn Leu Asn Asp
           435                 440                 445
   Gly Leu Trp His Ser Val Ser Ile Asn Ala Arg Arg Asn Arg Ile Thr
       450                 455                 460
   Leu Thr Leu Asp Asp Glu Ala Ala Pro Pro Ala Pro Asp Ser Thr Trp
   465                 470                 475                 480
   Val Gln Ile Tyr Ser Gly Asn Ser Tyr Tyr Phe Gly Gly Cys Pro Asp
                   485                 490                 495
   Asn Leu Thr Asp Ser Gln Cys Leu Asn Pro Ile Lys Ala Phe Gln Gly
               500                 505                 510
   Cys Met Arg Leu Ile Phe Ile Asp Asn Gln Pro Lys Asp Leu Ile Ser
           515                 520                 525
   Val Gln Gln Gly Ser Leu Gly Asn Phe Ser Asp Leu His Ile Asp Leu
       530                 535                 540
   Cys Ser Ile Lys Asp Arg Cys Leu Pro Asn Tyr Cys Glu His Gly Gly
   545                 550                 555                 560
   Ser Cys Ser Gln Ser Trp Thr Thr Phe Tyr Cys Asn Cys Ser Asp Thr
                   565                 570                 575
   Ser Tyr Thr Gly Ala Thr Cys His Asn Ser Ile Tyr Glu Gln Ser Cys
               580                 585                 590
   Glu Val Tyr Arg His Gln Gly Asn Thr Ala Gly Phe Phe Tyr Ile Asp
           595                 600                 605
   Ser Asp Gly Ser Gly Pro Leu Gly Pro Leu Gln Val Tyr Cys Asn Ile
       610                 615                 620
   Thr Glu Asp Lys Ile Trp Thr Ser Val Gln His Asn Asn Thr Glu Leu
   625                 630                 635                 640
   Thr Arg Val Arg Gly Ala Asn Pro Glu Lys Pro Tyr Ala Met Ala Leu
                   645                 650                 655
   Asp Tyr Gly Gly Ser Met Glu Gln Leu Glu Ala Val Ile Asp Gly Ser
               660                 665                 670
   Glu His Cys Glu Gln Glu Val Ala Tyr His Cys Arg Arg Ser Arg Leu
           675                 680                 685
   Leu Asn Thr Pro Asp Gly Thr Pro Phe Thr Trp Trp Ile Gly Arg Ser
       690                 695                 700
   Asn Glu Arg His Pro Tyr Trp Gly Gly Ser Pro Pro Gly Val Gln Gln
   705                 710                 715                 720
   Cys Glu Cys Gly Leu Asp Glu Ser Cys Leu Asp Ile Gln His Phe Cys
                   725                 730                 735
   Asn Cys Asp Ala Asp Lys Asp Glu Trp Thr Asn Asp Thr Gly Phe Leu
               740                 745                 750
   Ser Phe Lys Asp His Leu Pro Val Thr Gln Ile Val Ile Thr Asp Thr
           755                 760                 765
   Asp Arg Ser Asn Ser Glu Ala Ala Trp Arg Ile Gly Pro Leu Arg Cys
       770                 775                 780
   Tyr Gly Asp Arg Arg Phe Trp Asn Ala Val Ser Phe Tyr Thr Glu Ala
   785                 790                 795                 800
   Ser Tyr Leu His Phe Pro Thr Phe His Ala Glu Phe Ser Ala Asp Ile
                   805                 810                 815
   Ser Phe Phe Phe Lys Thr Thr Ala Leu Ser Gly Val Phe Leu Glu Asn
               820                 825                 830
   Leu Gly Ile Lys Asp Phe Ile Arg Leu Glu Ile Ser Ser Pro Ser Glu
           835                 840                 845
   Ile Thr Phe Ala Ile Asp Val Gly Asn Gly Pro Val Glu Leu Val Val
       850                 855                 860
   Gln Ser Pro Ser Leu Leu Asn Asp Asn Gln Trp His Tyr Val Arg Ala
   865                 870                 875                 880
   Glu Arg Asn Leu Lys Glu Thr Ser Leu Gln Val Asp Asn Leu Pro Arg
                   885                 890                 895
   Ser Thr Arg Glu Thr Ser Glu Glu Gly His Phe Arg Leu Gln Leu Asn
               900                 905                 910
   Ser Gln Leu Phe Val Gly Gly Thr Ser Ser Arg Gln Lys Gly Phe Leu
           915                 920                 925
   Gly Cys Ile Arg Ser Leu His Leu Asn Gly Gln Lys Met Asp Leu Glu
       930                 935                 940
   Glu Arg Ala Lys Val Thr Ser Gly Val Arg Pro Gly Cys Pro Gly His
   945                 950                 955                 960
   Cys Ser Ser Tyr Gly Ser Ile Cys His Asn Gly Gly Lys Cys Val Glu
                   965                 970                 975
   Lys His Asn Gly Tyr Leu Cys Asp Cys Thr Asn Ser Pro Tyr Glu Gly
               980                 985                 990
   Pro Phe Cys Lys Lys Glu Val Ser Ala Val Phe Glu Ala Gly Thr Ser
           995                 1000                1005
   Val Thr Tyr Met Phe Gln Glu Pro Tyr Pro Val Thr Lys Asn Ile Ser
       1010                1015                1020
   Leu Ser Ser Ser Ala Ile Tyr Thr Asp Ser Ala Pro Ser Lys Glu Asn
   1025                1030                1035                1040
   Ile Ala Leu Ser Phe Val Thr Thr Gln Ala Pro Ser Leu Leu Leu Phe
                   1045                1050                1055
   Ile Asn Ser Ser Ser Gln Asp Phe Val Val Val Leu Leu Cys Lys Asn
               1060                1065                1070
   Gly Ser Leu Gln Val Arg Tyr His Leu Asn Lys Glu Glu Thr His Val
           1075                1080                1085
   Phe Thr Ile Asp Ala Asp Asn Phe Ala Asn Arg Arg Met His His Leu
       1090                1095                1100
   Lys Ile Asn Arg Glu Gly Arg Glu Leu Thr Ile Gln Met Asp Gln Gln
   1105                1110                1115                1120
   Leu Arg Leu Ser Tyr Asn Phe Ser Pro Glu Val Glu Phe Arg Val Ile
                   1125                1130                1135
   Arg Ser Leu Thr Leu Gly Lys Val Thr Glu Asn Leu Gly Leu Asp Ser
               1140                1145                1150
   Glu Val Ala Lys Ala Asn Ala Met Gly Phe Ala Gly Cys Met Ser Ser
           1155                1160                1165
   Val Gln Tyr Asn His Ile Ala Pro Leu Lys Ala Ala Leu Arg His Ala
       1170                1175                1180
   Thr Val Ala Pro Val Thr Val His Gly Thr Leu Thr Glu Ser Ser Cys
   1185                1190                1195                1200
   Gly Phe Met Val Asp Ser Asp Val Asn Ala Val Thr Thr Val His Ser
                   1205                1210                1215
   Ser Ser Asp Pro Phe Gly Lys Thr Asp Glu Arg Glu Pro Leu Thr Asn
               1220                1225                1230
   Ala Val Arg Ser Asp Ser Ala Val Ile Gly Gly Val Ile Ala Val Val
           1235                1240                1245
   Ile Phe Ile Ile Phe Cys Ile Ile Gly Ile Met Thr Arg Phe Leu Tyr
       1250                1255                1260
   Gln His Lys Gln Ser His Arg Thr Ser Gln Met Lys Glu Lys Glu Tyr
   1265                1270                1275                1280
   Pro Glu Asn Leu Asp Ser Ser Phe Arg Asn Glu Ile Asp Leu Gln Asn
                   1285                1290                1295
   Thr Val Ser Glu Cys Lys Arg Glu Tyr Phe Ile
               1300                1305
<210> 3
   <211> 3780
   <212> DNA
   <213> homo sapiens
<400> 3
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac    360
   aaacaagaag acagcatctg gacctttgca ggaaacatga atgctgacag cgtggtgcac    420
   cacaagctat tgcactcagt gagagcccga tttgttcgct ttgtgcccct ggaatggaat    480
   cccagtggga agattggcat gagagtcgag gtctacggat gttcctataa atcagacgtt    540
   gctgactttg atggccgaag ctcacttctg tacaggttca atcagaagtt gatgagtact    600
   ctcaaagatg tgatctccct gaagttcaag agcatgcaag gagatggggt cctgttccat    660
   ggagaaggtc agcgtggaga ccacatcacc ttggaactcc agaaggggag gctcgcccta    720
   cacctcaatt tgggtgacag caaagcgcgg ctcagcagca gcttgccctc tgccaccctg    780
   ggcagcctcc tggatgacca gcactggcac tyggtcctca ttgagcgggt gggcaagcag    840
   gtgaacttca cggtggacaa gcacacacag cacttccgca ccaagggcga gacggatgcc    900
   ttagacattg actatgaggg caatgtcact ttttcctgct ccgaaccaca gattgtgccc    960
   atcacatttg tyaactccag cggcagctat ttgctgctgc ccggcacccc ccaaattgat   1020
   gggctctcag tgagtttcca gtttcgaaca tggaacaagg atggtctgct tctgtccaca   1080
   gagctgtctg agggctcggg aaccctgctg ctgagcctgg agggtggaat cctgagactc   1140
   ctgattcaga aaatgacaga acgcgtagct gaaatcctca caggcagcaa cttgaatgat   1200
   ggcctgtggc actcggttag catcaacgcc aggaggaacc gcatcacgct cactctggat   1260
   gatgaagcag cacccccggc tccagacagc acttgggtgc agatttattc tggaaatagc   1320
   tactattttg gagggtgccc cgacaatctc accgattccc aatgtttaaa tcccattaag   1380
   gctttccaag gctgcatgag gctcatcttt attgataacc agcccaagga cctcatttca   1440
   gttcagcaag gttccctggg gaattttagt gatttacaca ttgatctgtg tagcatcaaa   1500
   gacaggtgtt tgccaaacta ctgtgaacat ggaggaagct gctcccagtc ctggactacc   1560
   ttctattgta actgcagtga cacaagttac actggtgcca cctgccacaa ctccatctac   1620
   gagcaatcct gcgaggtgta caggcaccag gggaatacag ccggcttctt ctacatcgac   1680
   tcagatggca gcggcccact gggacctctc caggtgtact gcaatatcac tgaggacaag   1740
   atctggacat cagtgcagca caacaataca gagctgaccc gagtgcgggg cgctaaccct   1800
   gagaagccct atgccatggc cttggactac gggggcagca tggaacagct ggaggccgtg   1860
   atcgacggct ctgagcactg tgagcaggag gtggcctacc actgcaggag gtcccgcctg   1920
   ctcaacacgc cggatggaac accatttacc tggtggattg ggcggtccaa tgaaaggcac   1980
   ccttactggg gaggttcccc tcctggggtc cagcagtgtg agtgtggcct agacgagagc   2040
   tgcctggaca ttcagcactt ttgcaattgc gacgctgaca aggatgaatg gacaaatgat   2100
   actggctttc tttccttcaa agaccacttg cctgtcactc agatagttat cactgatacc   2160
   gacagatcaa actcagaagc cgcttggaga attggtccct tgcgttgcta tggtgaccga   2220
   cgcttctgga acgccgtctc attttataca gaagcctctt acctccactt tcctaccttc   2280
   catgcggaat tcagtgccga tatttccttc ttttttaaaa ccacagcatt atccggagtt   2340
   ttcctagaaa atcttggcat taaagacttc attcgactcg aaataagctc tccttcagag   2400
   atcacctttg ccatcgatgt tgggaatggt cctgtggagc ttgtagtcca gtctccttct   2460
   cttctgaatg acaaccaatg gcactatgtc cgggctgaga ggaacctcaa ggagacctcc   2520
   ctgcaggtgg acaaccttcc aaggagcacc agggagacgt cggaggaggg ccattttcga   2580
   ctgcagctga acagccagtt gtttgtaggg ggaacgtcat ccagacagaa aggcttccta   2640
   ggatgcattc gctccttaca cttgaatgga cagaaaatgg acctggaaga gagggcaaag   2700
   gtcacatctg gagtcaggcc aggctgcccc ggccactgca gcagctacgg cagcatctgc   2760
   cacaacgggg gcaagtgtgt ggagaagcac aatggctacc tgtgtgattg caccaattca   2820
   ccttatgaag ggcccttttg caaaaaagag gtttctgctg tttttgaggc tggcacgtcg   2880
   gttacttaca tgtttcaaga accctatcct gtgaccaaga atataagcct ctcatcctca   2940
   gctatttaca cagattcagc tccatccaag gaaaacattg cacttagctt tgtgacaacc   3000
   caggcaccca gtcttttgct ctttatcaat tcttcttctc aggacttcgt ggttgttctg   3060
   ctctgcaaga atggaagctt acaggttcgc tatcacctaa acaaggaaga aacccatgta   3120
   ttcaccattg atgcagataa ctttgctaac agaaggatgc accacttgaa gattaaccga   3180
   gagggaagag agcttaccat tcagatggac cagcaacttc gactcagtta taacttctct   3240
   ccggaagtag agttcagggt tataaggtca ctcaccttgg gcaaagtcac agagaatctt   3300
   ggtttggatt ctgaagttgc taaagcaaat gccatgggtt ttgctggatg catgtcttcc   3360
   gtccagtaca accacatagc accactgaag gctgccctgc gccatgccac tgtcgcgcct   3420
   gtgactgtcc atgggacctt gacggaatcc agctgtggct tcatggtgga ctcagatgtg   3480
   aatgcagtga ccacggtgca ttcttcatca gatccttttg ggaagacaga tgagcgggaa   3540
   ccactcacaa atgctgttcg aagtgattcg gcagtcatcg gaggggtgat agcagtggtg   3600
   atattcatca tcttctgtat catcggcatc atgacccggt tcctctacca gcacaagcag   3660
   tcacatcgta cgagccagat gaaggagaag gaatatccag aaaatttgga cagttccttc    3720
   agaaatgaaa ttgacttgca aaacacagtg agcgagtgta aacgggaata tttcatctga    3780
<210> 4
   <211> 1259
   <212> PRT
   <213> homo sapiens
<220>
   <221> VARIANT
   <222> (1)...(1259)
   <223> Xaa = Any Amino Acid
<400> 4
   Met Asp Ser Leu Pro Arg Leu Thr Ser Val Leu Thr Leu Leu Phe Ser
   1               5                   10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Gly Asn Val Thr Phe Ser Cys Ser Glu Pro Gln Ile Val Pro
   305                 310                 315                 320
   Ile Thr Phe Val Asn Ser Ser Gly Ser Tyr Leu Leu Leu Pro Gly Thr
                   325                 330                 335
   Pro Gln Ile Asp Gly Leu Ser Val Ser Phe Gln Phe Arg Thr Trp Asn
               340                 345                 350
   Lys Asp Gly Leu Leu Leu Ser Thr Glu Leu Ser Glu Gly Ser Gly Thr
           355                 360                 365
   Leu Leu Leu Ser Leu Glu Gly Gly Ile Leu Arg Leu Val Ile Gln Lys
       370                 375                 380
   Met Thr Glu Arg Val Ala Glu Ile Leu Thr Gly Ser Asn Leu Asn Asp
   385                 390                 395                 400
   Gly Leu Trp His Ser Val Ser Ile Asn Ala Arg Arg Asn Arg Ile Thr
                   405                 410                 415
   Leu Thr Leu Asp Asp Glu Ala Ala Pro Pro Ala Pro Asp Ser Thr Trp
               420                 425                 430
   Val Gln Ile Tyr Ser Gly Asn Ser Tyr Tyr Phe Gly Gly Cys Pro Asp
           435                 440                 445
   Asn Leu Thr Asp Ser Gln Cys Leu Asn Pro Ile Lys Ala Phe Gln Gly
       450                 455                 460
   Cys Met Arg Leu Ile Phe Ile Asp Asn Gln Pro Lys Asp Leu Ile Ser
   465                 470                 475                 480
   Val Gln Gln Gly Ser Leu Gly Asn Phe Ser Asp Leu His Ile Asp Leu
                   485                 490                 495
   Cys Ser Ile Lys Asp Arg Cys Leu Pro Asn Tyr Cys Glu His Gly Gly
               500                 505                 510
   Ser Cys Ser Gln Ser Trp Thr Thr Phe Tyr Cys Asn Cys Ser Asp Thr
           515                 520                 525
   Ser Tyr Thr Gly Ala Thr Cys His Asn Ser Ile Tyr Glu Gln Ser Cys
       530                 535                 540
   Glu Val Tyr Arg His Gln Gly Asn Thr Ala Gly Phe Phe Tyr Ile Asp
   545                 550                 555                 560
   Ser Asp Gly Ser Gly Pro Leu Gly Pro Leu Gln Val Tyr Cys Asn Ile
                   565                 570                 575
   Thr Glu Asp Lys Ile Trp Thr Ser Val Gln His Asn Asn Thr Glu Leu
               580                 585                 590
   Thr Arg Val Arg Gly Ala Asn Pro Glu Lys Pro Tyr Ala Met Ala Leu
           595                 600                 605
   Asp Tyr Gly Gly Ser Met Glu Gln Leu Glu Ala Val Ile Asp Gly Ser
       610                 615                 620
   Glu His Cys Glu Gln Glu Val Ala Tyr His Cys Arg Arg Ser Arg Leu
   625                 630                 635                 640
   Leu Asn Thr Pro Asp Gly Thr Pro Phe Thr Trp Trp Ile Gly Arg Ser
                   645                 650                 655
   Asn Glu Arg His Pro Tyr Trp Gly Gly Ser Pro Pro Gly Val Gln Gln
               660                 665                 670
   Cys Glu Cys Gly Leu Asp Glu Ser Cys Leu Asp Ile Gln His Phe Cys
           675                 680                 685
   Asn Cys Asp Ala Asp Lys Asp Glu Trp Thr Asn Asp Thr Gly Phe Leu
       690                 695                 700
   Ser Phe Lys Asp His Leu Pro Val Thr Gln Ile Val Ile Thr Asp Thr
   705                 710                 715                 720
   Asp Arg Ser Asn Ser Glu Ala Ala Trp Arg Ile Gly Pro Leu Arg Cys
                   725                 730                 735
   Tyr Gly Asp Arg Arg Phe Trp Asn Ala Val Ser Phe Tyr Thr Glu Ala
               740                 745                 750
   Ser Tyr Leu His Phe Pro Thr Phe His Ala Glu Phe Ser Ala Asp Ile
           755                 760                 765
   Ser Phe Phe Phe Lys Thr Thr Ala Leu Ser Gly Val Phe Leu Glu Asn
       770                 775                 780
   Leu Gly Ile Lys Asp Phe Ile Arg Leu Glu Ile Ser Ser Pro Ser Glu
   785 790 795 800
   Ile Thr Phe Ala Ile Asp Val Gly Asn Gly Pro Val Glu Leu Val Val
   805 810 815
   Gln Ser Pro Ser Leu Leu Asn Asp Asn Gln Trp His Tyr Val Arg Ala
   820 825 830
   Glu Arg Asn Leu Lys Glu Thr Ser Leu Gln Val Asp Asn Leu Pro Arg
   835 840 845
   Ser Thr Arg Glu Thr Ser Glu Glu Gly His Phe Arg Leu Gln Leu Asn
   850 855 860
   Ser Gln Leu Phe Val Gly Gly Thr Ser Ser Arg Gln Lys Gly Phe Leu
   865 870 875 880
   Gly Cys Ile Arg Ser Leu His Leu Asn Gly Gln Lys Met Asp Leu Glu
   885 890 895
   Glu Arg Ala Lys Val Thr Ser Gly Val Arg Pro Gly Cys Pro Gly His
   900 905 910
   Cys Ser Ser Tyr Gly Ser Ile Cys His Asn Gly Gly Lys Cys Val Glu
   915 920 925
   Lys His Asn Gly Tyr Leu Cys Asp Cys Thr Asn Ser Pro Tyr Glu Gly
   930 935 940
   Pro Phe Cys Lys Lys Glu Val Ser Ala Val Phe Glu Ala Gly Thr Ser
   945 950 955 960
   Val Thr Tyr Met Phe Gln Glu Pro Tyr Pro Val Thr Lys Asn Ile Ser
   965 970 975
   Leu Ser Ser Ser Ala Ile Tyr Thr Asp Ser Ala Pro Ser Lys Glu Asn
   980 985 990
   Ile Ala Leu Ser Phe Val Thr Thr Gln Ala Pro Ser Leu Leu Leu Phe
   995 1000 1005
   Ile Asn Ser Ser Ser Gln Asp Phe Val Val Val Leu Leu Cys Lys Asn
   1010 1015 1020
   Gly Ser Leu Gln Val Arg Tyr His Leu Asn Lys Glu Glu Thr His Val
   1025 1030 1035 1040
   Phe Thr Ile Asp Ala Asp Asn Phe Ala Asn Arg Arg Met His His Leu
   1045 1050 1055
   Lys Ile Asn Arg Glu Gly Arg Glu Leu Thr Ile Gln Met Asp Gln Gln
   1060 1065 1070
   Leu Arg Leu Ser Tyr Asn Phe Ser Pro Glu Val Glu Phe Arg Val Ile
   1075 1080 1085
   Arg Ser Leu Thr Leu Gly Lys Val Thr Glu Asn Leu Gly Leu Asp Ser
   1090 1095 1100
   Glu Val Ala Lys Ala Asn Ala Met Gly Phe Ala Gly Cys Met Ser Ser
   1105 1110 1115 1120
   Val Gln Tyr Asn His Ile Ala Pro Leu Lys Ala Ala Leu Arg His Ala
   1125 1130 1135
   Thr Val Ala Pro Val Thr Val His Gly Thr Leu Thr Glu Ser Ser Cys
   1140 1145 1150
   Gly Phe Met Val Asp Ser Asp Val Asn Ala Val Thr Thr Val His Ser
   1155 1160 1165
   Ser Ser Asp Pro Phe Gly Lys Thr Asp Glu Arg Glu Pro Leu Thr Asn
   1170 1175 1180
   Ala Val Arg Ser Asp Ser Ala Val Ile Gly Gly Val Ile Ala Val Val
   1185 1190 1195 1200
   Ile Phe Ile Ile Phe Cys Ile Ile Gly Ile Met Thr Arg Phe Leu Tyr
   1205 1210 1215
   Gln His Lys Gln Ser His Arg Thr Ser Gln Met Lys Glu Lys Glu Tyr
   1220 1225 1230
   Pro Glu Asn Leu Asp Ser Ser Phe Arg Asn Glu Ile Asp Leu Gln Asn
           1235                1240                1245
   Thr Val Ser Glu Cys Lys Arg Glu Tyr Phe Ile
       1250                1255
<210> 5
   <211> 108
   <212> DNA
   <213> homo sapiens
<400> 5
   atggattctt taccacggct gaccagcgtt ttgactttgc tgttctctgg cttgtggcat      60
   ttaggattaa cagcgacaaa ctacctttgc aggaaacatg aatgctga                   108
<210> 6
   <211> 35
   <212> PRT
   <213> homo sapiens
<400> 6
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Leu Cys Arg Lys
               20                  25                  30
   His Glu Cys
           35
<210> 7
   <211> 753
   <212> DNA
   <213> homo sapiens
<400> 7
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaagctat tgcactcagt gagagcccga tttgttcgct ttgtgcccct ggaatggaat     480
   cccagtggga agattggcat gagagtcgag gtctacggat gttcctataa atcagacgtt     540
   gctgactttg atggccgaag ctcacttctg tacaggttca atcagaagtt gatgagtact     600
   ctcaaagatg tgatctccct gaagttcaag agcatgcaag gagatggggt cctgttccat     660
   ggagaaggtc agcgtggaga ccacatcacc ttggaactcc agaaggggag gctcgcccta     720
   cacctcaatt tggtggtttg tagttctcct tga                                  753
<210> 8
   <211> 250
   <212> PRT
   <213> homo sapiens
<400> 8
   Met Asp Ser Leu Pro Arg Leu Thr Ser Val Leu Thr Leu Leu Phe Ser
    1              5                   10                  15
   Gly Leu Trp His Leu Gly Leu Thr Ala Thr Asn Tyr Asn Cys Asp Asp
                   20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Val Val Cys Ser Ser Pro
                   245                 250
<210> 9
   <211> 840
   <212> DNA
   <213> homo sapiens
<400> 9
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaagctat tgcactcagt gagagcccga tttgttcgct ttgtgcccct ggaatggaat     480
   cccagtggga agattggcat gagagtcgag gtctacggat gttcctataa atcagacgtt     540
   gctgactttg atggccgaag ctcacttctg tacaggttca atcagaagtt gatgagtact     600
   ctcaaagatg tgatctccct gaagttcaag agcatgcaag gagatggggt cctgttccat     660
   ggagaaggtc agcgtggaga ccacatcacc ttggaactcc agaaggggag gctcgcccta     720
   cacctcaatt tgggtgacag caaagcgcgg ctaagcactt gccctctgcc accctgggca     780
   gcctcctgga tgaccagcac tggcactygg tcctcattga gcgggtgggc aagcaggtga     840
<210> 10
   <211> 279
   <212> PRT
   <213> homo sapiens
<220>
   <221> VARIANT
   <222> (1)...(279)
   <223> Xaa = Any Amino Acid
<400> 10
   Met Asp Ser Leu Pro Arg Leu Thr Ser Val Leu Thr Leu Leu Phe Ser
   1               5                   10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Thr Cys Pro Leu
                   245                 250                 255
   Pro Pro Trp Ala Ala Ser Trp Met Thr Ser Thr Gly Thr Xaa Ser Ser
               260                 265                 270
   Leu Ser Gly Trp Ala Ser Arg
           275
<210> 11
   <211> 1749
   <212> DNA
   <213> homo sapiens
<400> 11
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaagctat tgcactcagt gagagcccga tttgttcgct ttgtgcccct ggaatggaat    480
   cccagtggga agattggcat gagagtcgag gtctacggat gttcctataa atcagacgtt    540
   gctgactttg atggccgaag ctcacttctg tacaggttca atcagaagtt gatgagtact    600
   ctcaaagatg tgatctccct gaagttcaag agcatgcaag gagatggggt cctgttccat    660
   ggagaaggtc agcgtggaga ccacatcacc ttggaactcc agaaggggag gctcgcccta    720
   cacctcaatt tgggtgacag caaagcgcgg ctcagcagca gcttgccctc tgccaccctg    780
   ggcagcctcc tggatgacca gcactggcac tyggtcctca ttgagcgggt gggcaagcag    840
   gtgaacttca cggtggacaa gcacacacag cacttccgca ccaagggcga gacggatgcc    900
   ttagacattg actatgagct tagttttgga ggaattccag taccaggaaa acctgggacc    960
   tttttaaaga aaaacttcca tggatgcatc gaaaaccttt actacaatgg agtaaacata   1020
   atttacctgg ctaagagacg aaagcatcag atctatactg tgggcaatgt cactttttcc   1080
   tgctccgaac cacagattgt gcccatcaca tttgtyaact ccagcggcag ctatttgctg   1140
   ctgcccggca ccccccaaat tgatgggctc tcagtgagtt tccagtttcg aacatggaac   1200
   aaggatggtc tgcttctgtc cacagagctg tctgagggct cgggaaccct gctgctgagc   1260
   ctggagggtg gaatcctgag actcgtgatt cagaaaatga cagaacgcgt agctgaaatc   1320
   ctcacaggca gcaacttgaa tgatggcctg tggcactcgg ttagcatcaa cgccaggagg   1380
   aaccgcatca cgctcactct ggatgatgaa gcagcacccc cggctccaga cagcacttgg   1440
   gtgcagattt attctggaaa tagctactat tttggaggtg tttgccaaac tactgtgaac   1500
   atggaggaag ctgctcccag tcctggacta ccttctattg taactgcagt gacacaagtt   1560
   acactggtgc cacctgccac aactccatct acgagcaatc ctgcgaggtg tacaggcacc   1620
   aggggaatac agccggcttc ttctacatcg actcagatgg cagcggccca ctgggacctc   1680
   tccaggtgta ctgcaatatc actgaggaca agatctggac atcagtgcag cacaacaata   1740
   cagagctga                                                           1749
<210> 12
   <211> 582
   <212> PRT
   <213> homo sapiens
<220>
   <221> VARIANT
   <222> (1)...(582)
   <223> Xaa = Any Amino Acid
<400> 12
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                           165                 170                 175
   Lys Ser Asp Val Ala Asp Phe Asp Gly Arg Ser Ser Leu Leu Tyr Arg
                   180                 185                 190
   Phe Asn Gln Lys Leu Met Ser Thr Leu Lys Asp Val Ile Ser Leu Lys
               195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Leu Ser Phe Gly Gly Ile Pro Val Pro Gly Lys Pro Gly Thr
   305                 310                 315                 320
   Phe Leu Lys Lys Asn Phe His Gly Cys Ile Glu Asn Leu Tyr Tyr Asn
                   325                 330                 335
   Gly Val Asn Ile Ile Xaa Leu Ala Lys Arg Arg Lys His Gln Ile Tyr
               340                 345                 350
   Thr Val Gly Asn Val Thr Phe Ser Cys Ser Glu Pro Gln Ile Val Pro
           355                 360                 365
   Ile Thr Phe Val Asn Ser Ser Gly Ser Tyr Leu Leu Leu Pro Gly Thr
       370                 375                 380
   Pro Gln Ile Asp Gly Leu Ser Val Ser Phe Gln Phe Arg Thr Trp Asn
   385                 390                 395                 400
   Lys Asp Gly Leu Leu Leu Ser Thr Glu Leu Ser Glu Gly Ser Gly Thr
                   405                 410                 415
   Leu Leu Leu Ser Leu Glu Gly Gly Ile Leu Arg Leu Val Ile Gln Lys
               420                 425                 430
   Met Thr Glu Arg Val Ala Glu Ile Leu Thr Gly Ser Asn Leu Asn Asp
           435                 440                 445
   Gly Leu Trp His Ser Val Ser Ile Asn Ala Arg Arg Asn Arg Ile Thr
       450                 455                 460
   Leu Thr Leu Asp Asp Glu Ala Ala Pro Pro Ala Pro Asp Ser Thr Trp
   465                 470                 475                 480
   Val Gln Ile Tyr Ser Gly Asn Ser Tyr Tyr Phe Gly Gly Val Cys Gln
                   485                 490                 495
   Thr Thr Val Asn Met Glu Glu Ala Ala Pro Ser Pro Gly Leu Pro Ser
               500                 505                 510
   Ile Val Thr Ala Val Thr Gln Val Thr Leu Val Pro Pro Ala Thr Thr
           515                 520                 525
   Pro Ser Thr Ser Asn Pro Ala Arg Cys Thr Gly Thr Arg Gly Ile Gln
       530                 535                 540
   Pro Ala Ser Ser Thr Ser Thr Gln Met Ala Ala Ala His Trp Asp Leu
   545                 550                 555                 560
   Ser Arg Cys Thr Ala Ile Ser Leu Arg Thr Arg Ser Gly His Gln Cys
                   565                 570                 575
   Ser Thr Thr Ile Gln Ser
               580
<210> 13
   <211> 1605
   <212> DNA
   <213> homo sapiens
<400> 13
   atggattcct taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaaactat tgcactcagt gagagcccga tttgttcgct ttgtgcccct ggaatggaat     480
   cccagtggaa agattggcat gagagtcgag gtctacggat gttcctataa atcagacgtt     540
   gctgactttc atggccgaag ctcacttctg tacaggttca atcagaagtt gatgagtact     600
   ctcaaagatg tgatctccct gaagttcaag agcatgcaag gagatggggt cctgttccat     660
   ggagaaggtc agcgtggaga ccacatcacc ttggaactcc agaaggggag gctcgcccta     720
   cacctcaatt tgggtgacag caaagcgcgg ctcagcagca gcttgccctc tgccaccctg     780
   ggcagcctcc tggatgacca gcactggcac tyggtcctca ttgagcgggt gggcaagcag     840
   gtgaacttca cggtggacaa gcacacacag cacttccgca ccaagggcga gacggatgcc     900
   ttagacattg actatgaggg caatgtcact ttttcctgct ccgaaccaca gattgtgccc     960
   atcacatttg tyaactccag cggcagctat ttgctgctgc ccggcacccc ccaaattgat    1020
   gggctctcag tgagtttcca gtttcgaaca tggaacaagg atggtctgct tctgtccaca    1080
   gagctgtctg agggctcggg aaccctgctg ctgagcctgg agggtggaat cctgagactc    1140
   gtgattcaga aaatgacaga acgcgtagct gaaatcctca caggcagcaa cttgaatgat    1200
   ggcctgtggc actcggttag catcaacgcc aggaggaacc gcatcacgct cactctggat    1260
   gatgaagcag cacccccggc tccagacagc acttgggtgc agatttattc tggaaatagc    1320
   tactattttg gaggtgtttg ccaaactact gtgaacatgg aggaagctgc tcccagtcct    1380
   ggactacctt ctattgtaac tgcagtgaca caagttacac tggtgccacc tgccacaact    1440
   ccatctacga gcaatcctgc gaggtgtaca ggcaccaggg gaatacagcc ggcttcttct    1500
   acatcgactc agatggcagc ggcccactgg gacctctcca ggtgtactgc aatatcactg    1560
   aggacaagat ctggacatca gtgcagcaca acaatacaga gctga                     1605
<210> 14
   <211> 534
   <212> PRT
   <213> homo sapiens
<220>
   <221> VARIANT
   <222> (1)...(534)
   <223> Xaa = Any Amino Acid
<400> 14
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Gly Asn Val Thr Phe Ser Cys Ser Glu Pro Gln Ile Val Pro
   305                 310                 315                 320
   Ile Thr Phe Val Asn Ser Ser Gly Ser Tyr Leu Leu Leu Pro Gly Thr
                   325                 330                 335
   Pro Gln Ile Asp Gly Leu Ser Val Ser Phe Gln Phe Arg Thr Trp Asn
               340                 345                 350
   Lys Asp Gly Leu Leu Leu Ser Thr Glu Leu Ser Glu Gly Ser Gly Thr
           355                 360                 365
   Leu Leu Leu Ser Leu Glu Gly Gly Ile Leu Arg Leu Val Ile Gln Lys
       370                 375                 380
   Met Thr Glu Arg Val Ala Glu Ile Leu Thr Gly Ser Asn Leu Asn Asp
   385                 390                 395                 400
   Gly Leu Trp His Ser Val Ser Ile Asn Ala Arg Arg Asn Arg Ile Thr
                   405                 410                 415
   Leu Thr Leu Asp Asp Glu Ala Ala Pro Pro Ala Pro Asp Ser Thr Trp
               420                 425                 430
   Val Gln Ile Tyr Ser Gly Asn Ser Tyr Tyr Phe Gly Gly Val Cys Gln
           435                 440                 445
   Thr Thr Val Asn Met Glu Glu Ala Ala Pro Ser Pro Gly Leu Pro Ser
       450                 455                 460
   Ile Val Thr Ala Val Thr Gln Val Thr Leu Val Pro Pro Ala Thr Thr
   465                 470                 475                 480
   Pro Ser Thr Ser Asn Pro Ala Arg Cys Thr Gly Thr Arg Gly Ile Gln
                   485                 490                 495
   Pro Ala Ser Ser Thr Ser Thr Gln Met Ala Ala Ala His Trp Asp Leu
               500                 505                 510
   Ser Arg Cys Thr Ala Ile Ser Leu Arg Thr Arg Ser Gly His Gln Cys
           515                 520                 525
   Ser Thr Thr Ile Gln Ser
       530
<210> 15
   <211> 2238
   <212> DNA
   <213> homo sapiens
<400> 15
   atggattctt taccacggct gaccagcgtt ttgactttgc tgttctctgg cttgtggcat     60
   ttaggattaa cagcgacaaa ctacaactgt gatgatccac tagcatccct gctctctcca    120
   atggcttttt ccagttcctc agacctcact ggcactcaca gcccagctca actcaactgg    180
   agagttggaa ctggcggttg gtccccagca gattccaatg ctcaacagtg gctccagatg    240
   gacctgggaa acagagtaga gattacagca gtggccacgc agggaagata cggaagctct    300
   gactgggtga cgagttacag cctgatgttc agtgacacag gacgcaactg gaaacagtac    360
   aaacaagaag acagcatctg gacctttgca ggaaacatga atgctgacag cgtggtgcac    420
   cacaagctat tgcactcagt gagagcccga tttgttcgct ttgtgcccct ggaatggaat    480
   cccagtggga agattggcat gagagtcgag gtctacggat gttcctataa atcagacgtt    540
   gctgactttg atggccgaag ctcacttctg tacaggttca atcagaagtt gatgagtact    600
   ctcaaagatg tgatctccct gaagttcaag agcatgcaag gagatggggt cctgttccat    660
   ggagaaggtc agcgtggaga ccacatcacc ttggaactcc agaaggggag gctcgcccta    720
   cacctcaatt tgggtgacag caaagcgcgg ctcagcagca gcttgccctc tgccaccctg    780
   ggcagcctcc tggatgacca gcactggcac tyggtcctca ttgagcgggt gggcaagcag    840
   gtgaacttca cggtggacaa gcacacacag cacttccgca ccaagggcga gacggatgcc    900
   ttagacattg actatgagct tagttttgga ggaattccag taccaggaaa acctgggacc    960
   tttttaaaga aaaacttcca tggatgcatc gaaaaccttt actacaatgg agtaaacata   1020
   attracctgg ctaagagacg aaagcatcag atctatactg tgggcaatgt cactttttcc   1080
   tgctccgaac cacagattgt gcccatcaca tttgtyaact ccagcggcag ctatttgctg   1140
   ctgcccggca ccccccaaat tgatgggctc tcagtgagtt tccagtttcg aacatggaac   1200
   aaggatggtc tgcttctgtc cacagagctg tctgagggct cgggaaccct gctgctgagc   1260
   ctggagggtg gaatcctgag actcgtgatt cagaaaatga cagaacgcgt agctgaaatc   1320
   ctcacaggca gcaacttgaa tgatggcctg tggcactcgg ttagcatcaa cgccaggagg   1380
   aaccgcatca cgctcactct ggatgatgaa gcagcacccc cggctccaga cagcacttgg   1440
   gtgcagattt attctggaaa tagctactat tttggagggt gccccgacaa tctcaccgat   1500
   tcccaatgtt taaatcccat taaggctttc caaggctgca tgaggctcat ctttattgat   1560
   aaccagccca aggacctcat ttcagttcag caaggttccc tggggaattt tagtgattta   1620
   cacattgatc tgtgtagcat caaagacagg tgtttgccaa actactgtga acatggagga   1680
   agctgctccc agtcctggac taccttctat tgtaactgca gtgacacaag ttacactggt   1740
   gccacctgcc acaactccat ctacgagcaa tcctgcgagg tgtacaggca ccaggggaat   1800
   acagccggct tcttctacat cgactcagat ggcagcggcc cactgggacc tctccaggtg   1860
   tactgcaata tcactgagga caagatctgg acatcagtgc agcacaacaa tacagagctg   1920
   acccgagtgc ggggcgctaa ccctgagaag ccctatgcca tggccttgga ctacgggggc   1980
   agcatggaac agctggaggc cgtgatcgac ggctctgagc actgtgagca ggaggtggcc   2040
   taccactgca ggaggtcccg cctgctcaac acgccggatg gaacaccatt tacctggtgg   2100
   attgggcggt ccaatgaaag gcacccttac tggggaggtt cccctcctgg ggtccagcag   2160
   tgtgagtgtg gcctagacga gagctgcctg gacattcagc acttttgcaa ttgcgacgct   2220
   gacaaggatg aatggtaa                                                 2238
<210> 16
   <211> 745
   <212> PRT
   <213> homo sapiens
<220>
   <221> VARIANT
   <222> (1)...(745)
   <223> Xaa = Any Amino Acid
<400> 16
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Leu Ser Phe Gly Gly Ile Pro Val Pro Gly Lys Pro Gly Thr
   305                 310                 315                 320
   Phe Leu Lys Lys Asn Phe His Gly Cys Ile Glu Asn Leu Tyr Tyr Asn
                   325                 330                 335
   Gly Val Asn Ile Ile Xaa Leu Ala Lys Arg Arg Lys His Gln Ile Tyr
               340                 345                 350
   Thr Val Gly Asn Val Thr Phe Ser Cys Ser Glu Pro Gln Ile Val Pro
           355                 360                 365
   Ile Thr Phe Val Asn Ser Ser Gly Ser Tyr Leu Leu Leu Pro Gly Thr
       370                 375                 380
   Pro Gln Ile Asp Gly Leu Ser Val Ser Phe Gln Phe Arg Thr Trp Asn
   385                 390                 395                 400
   Lys Asp Gly Leu Leu Leu Ser Thr Glu Leu Ser Glu Gly Ser Gly Thr
                   405                 410                 415
   Leu Leu Leu Ser Leu Glu Gly Gly Ile Leu Arg Leu Val Ile Gln Lys
               420                 425                 430
   Met Thr Glu Arg Val Ala Glu Ile Leu Thr Gly Ser Asn Leu Asn Asp
           435                 440                 445
   Gly Leu Trp His Ser Val Ser Ile Asn Ala Arg Arg Asn Arg Ile Thr
   450 455 460
   Leu Thr Leu Asp Asp Glu Ala Ala Pro Pro Ala Pro Asp Ser Thr Trp
   465 470 475 480
   Val Gln Ile Tyr Ser Gly Asn Ser Tyr Tyr Phe Gly Gly Cys Pro Asp
   485 490 495
   Asn Leu Thr Asp Ser Gln Cys Leu Asn Pro Ile Lys Ala Phe Gln Gly
   500 505 510
   Cys Met Arg Leu Ile Phe Ile Asp Asn Gln Pro Lys Asp Leu Ile Ser
   515 520 525
   Val Gln Gln Gly Ser Leu Gly Asn Phe Ser Asp Leu His Ile Asp Leu
   530 535 540
   Cys Ser Ile Lys Asp Arg Cys Leu Pro Asn Tyr Cys Glu His Gly Gly
   545 550 555 560
   Ser Cys Ser Gln Ser Trp Thr Thr Phe Tyr Cys Asn Cys Ser Asp Thr
   565 570 575
   Ser Tyr Thr Gly Ala Thr Cys His Asn Ser Ile Tyr Glu Gln Ser Cys
   580 585 590
   Glu Val Tyr Arg His Gln Gly Asn Thr Ala Gly Phe Phe Tyr Ile Asp
   595 600 605
   Ser Asp Gly Ser Gly Pro Leu Gly Pro Leu Gln Val Tyr Cys Asn Ile
   610 615 620
   Thr Glu Asp Lys Ile Trp Thr Ser Val Gln His Asn Asn Thr Glu Leu
   625 630 635 640
   Thr Arg Val Arg Gly Ala Asn Pro Glu Lys Pro Tyr Ala Met Ala Leu
   645 650 655
   Asp Tyr Gly Gly Ser Met Glu Gln Leu Glu Ala Val Ile Asp Gly Ser
   660 665 670
   Glu His Cys Glu Gln Glu Val Ala Tyr His Cys Arg Arg Ser Arg Leu
   675 680 685
   Leu Asn Thr Pro Asp Gly Thr Pro Phe Thr Trp Trp Ile Gly Arg Ser
   690 695 700
   Asn Glu Arg His Pro Tyr Trp Gly Gly Ser Pro Pro Gly Val Gln Gln
   705 710 715 720
   Cys Glu Cys Gly Leu Asp Glu Ser Cys Leu Asp Ile Gln His Phe Cys
   725 730 735
   Asn Cys Asp Ala Asp Lys Asp Glu Trp
   740 745
<210> 17
   <211> 2094
   <212> DNA
   <213> homo sapiens
<400> 17
   atggattctt taccacggct gaccagcgtt ttgactttgc tgttctctgg cttgtggcat      60
   ttaggattaa cagcgacaaa ctacaactgt gatgatccac tagcatccct gctctctcca     120
   atggcttttt ccagttcctc agacctcact ggcactcaca gcccagctca actcaactgg     180
   agagttggaa ctggcggttg gtccccagca gattccaatg ctcaacagtg gctccagatg     240
   gacctgggaa acagagtaga gattacagca gtggccacgc agggaagata cggaagctct     300
   gactgggtga cgagttacag cctgatgttc agtgacacag gacgcaactg gaaacagtac     360
   aaacaagaag acagcatctg gacctttgca ggaaacatga atgctgacag cgtggtgcac     420
   cacaagctat tgcactcagt gagagcccga tttgttcgct ttgtgcccct ggaatggaat     480
   cccagtggga agattggcat gagagtcgag gtctacggat gttcctataa atcagacgtt     540
   gctgactttg atggccgaag ctcacttctg tacaggttca atcagaagtt gatgagtact     600
   ctcaaagatg tgatctccct gaagttcaag agcatgcaag gagatggggt cctgttccat     660
   ggagaaggtc agcgtggaga ccacatcacc ttggaactcc agaaggggag gctcgcccta     720
   cacctcaatt tgggtgacag caaagcgcgg ctcagcagca gcttgccctc tgccaccctg     780
   ggcagcctcc tggatgacca gcactggcac tyggtcctca ttgagcgggt gggcaagcag     840
   gtgaacttca cggtggacaa gcacacacag cacttccgca ccaagggcga gacggatgcc     900
   ttagacattg actatgaggg caatgtcact ttttcctgct ccgaaccaca gattgtgccc     960
   atcacatttg tyaactccag cggcagctat ttgctgctgc ccggcacccc ccaaattgat    1020
   gggctctcag tgagtttcca gtttcgaaca tggaacaagg atggtctgct tctgtccaca    1080
   gagctgtctg agggctcggg aaccctgctg ctgagcctgg agggtggaat cctgagactc    1140
   gtgattcaga aaatgacaga acgcgtagct gaaatcctca caggcagcaa cttgaatgat    1200
   ggcctgtggc actcggttag catcaacgcc aggaggaacc gcatcacgct cactctggat    1260
   gatgaagcag cacccccggc tccagacagc acttgggtgc agatttattc tggaaatagc    1320
   tactattttg gagggtgccc cgacaatctc accgattccc aatgtttaaa tcccattaag    1380
   gctttccaag gctgcatgag gctcatcttt attgataacc agcccaagga cctcatttca    1440
   gttcagcaag gttccctggg gaattttagt gatttacaca ttgatctgtg tagcatcaaa    1500
   gacaggtgtt tgccaaacta ctgtgaacat ggaggaagct gctcccagtc ctggactacc    1560
   ttctattgta actgcagtga cacaagttac actggtgcca cctgccacaa ctccatctac    1620
   gagcaatcct gcgaggtgta caggcaccag gggaatacag ccggcttctt ctacatcgac    1680
   tcagatggca gcggcccact gggacctctc caggtgtact gcaatatcac tgaggacaag    1740
   atctggacat cagtgcagca caacaataca gagctgaccc gagtgcgggg cgctaaccct    1800
   gagaagccct atgccatggc cttggactac gggggcagca tggaacagct ggaggccgtg    1860
   atcgacggct ctgagcactg tgagcaggag gtggcctacc actgcaggag gtcccgcctg    1920
   ctcaacacgc cggatggaac accatttacc tggtggattg ggcggtccaa tgaaaggcac    1980
   ccttactggg gaggttcccc tcctggggtc cagcagtgtg agtgtggcct agacgagagc    2040
   tgcctggaca ttcagcactt ttgcaattgc gacgctgaca aggatgaatg gtaa          2094
<213> 18
   <211> 697
   <212> PRT
   <213> homo sapiens
<220>
   <221> VARIANT
   <222> (1)...(697)
   <223> Xaa = Any Amino Acid
<400> 18
<210> 19
   <211> 2520
   <211> DNA
   <213> homo sapiens
<400> 19
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ttagacattg actatgagct tagttttgga ggaattccag taccaggaaa acctgggacc 960
   tttttaaaga aaaacttcca tggatgcatc gaaaaccttt actacaatgg agtaaacata 1020
   attracctgg ctaagagacg aaagcatcag atctatactg tgggcaatgt cactttttcc 1080
   tgctccgaac cacagattgt gcccatcaca tttgtyaact ccagcggcag ctatttgctg 1140
   ctgcccggca ccccccaaat tgatgggctc tcagtgagtt tccagtttcg aacatggaac 1200
   aaggatggtc tgcttctgtc cacagagctg tctgagggct cgggaaccct gctgctgagc 1260
   ctggagggtg gaatcctgag actcgtgatt cagaaaatga cagaacgcgt agctgaaatc 1320
   ctcacaggca gcaacttgaa tgatggcctg tggcactcgg ttagcatcaa cgccaggagg 1380
   aaccgcatca cgctcactct ggatgatgaa gcagcacccc cggctccaga cagcacttgg 1440
   gtgcagattt attctggaaa tagctactat tttggagggt gccccgacaa tctcaccgat 1500
   tcccaatgtt taaatcccat taaggctttc caaggctgca tgaggctcat ctttattgat 1560
   aaccagccca aggacctcat ttcagttcag caaggttccc tggggaattt tagtgattta 1620
   cacattgatc tgtgtagcat caaagacagg tgtttgccaa actactgtga acatggagga 1680
   agctgctccc agtcctggac taccttctat tgtaactgca gtgacacaag ttacactggt 1740
   gccacctgcc acaactccat ctacgagcaa tcctgcgagg tgtacaggca ccaggggaat 1800
   acagccggct tcttctacat cgactcagat ggcagcggcc cactgggacc tctccaggtg 1860
   tactgcaata tcactgagga caagatctgg acatcagtgc agcacaacaa tacagagctg 1920
   acccgagtgc ggggcgctaa ccctgagaag ccctatgcca tggccttgga ctacgggggc 1980
   agcatggaac agctggaggc cgtgatcgac ggctctgagc actgtgagca ggaggtggcc 2040
   taccactgca ggaggtcccg cctgctcaac acgccggatg gaacaccatt tacctggtgg 2100
   attgggcggt ccaatgaaag gcacccttac tggggaggtt cccctcctgg ggtccagcag 2160
   tgtgagtgtg gcctagacga gagctgcctg gacattcagc acttttgcaa ttgcgacgct 2220
   gacaaggatg aatggacaaa tgatactggc tttctttcct tcaaagacca cttgcctgtc    2280
   actcagatag ttatcactga taccgacaga tcaaactcag aagccgcttg gagaattggt    2340
   cccttgcgtt gctatggtga ccgtgagtac aaaatcgaaa gaagctttct ctctgcatta    2400
   catgagcaca agatgttctt actcccttat cccttttccc tgcagtgtgc cctagtcttg    2460
   aaaattatcc acatgtccag tgctttccca taccccactg aaaacgataa accatgttga    2520
<210> 20
   <211> 839
   <212> PRT
   <213> homo sapiens
<220>
   <221> VARIANT
   <222> (1)...(839)
   <223> Xaa = Any Amino Acid
<400> 20
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Leu Ser Phe Gly Gly Ile Pro Val Pro Gly Lys Pro Gly Thr
   305                 310                 315                 320
   Phe Leu Lys Lys Asn Phe His Gly Cys Ile Glu Asn Leu Tyr Tyr Asn
                   325                 330                 335
   Gly Val Asn Ile Ile Xaa Leu Ala Lys Arg Arg Lys His Gln Ile Tyr
               340                 345                 350
   Thr Val Gly Asn Val Thr Phe Ser Cys Ser Glu Pro Gln Ile Val Pro
           355                 360                 365
   Ile Thr Phe Val Asn Ser Ser Gly Ser Tyr Leu Leu Leu Pro Gly Thr
       370                 375                 380
   Pro Gln Ile Asp Gly Leu Ser Val Ser Phe Gln Phe Arg Thr Trp Asn
   385                 390                 395                 400
   Lys Asp Gly Leu Leu Leu Ser Thr Glu Leu Ser Glu Gly Ser Gly Thr
                   405                 410                 415
   Leu Leu Leu Ser Leu Glu Gly Gly Ile Leu Arg Leu Val Ile Gln Lys
               420                 425                 430
   Met Thr Glu Arg Val Ala Glu Ile Leu Thr Gly Ser Asn Leu Asn Asp
           435                 440                 445
   Gly Leu Trp His Ser Val Ser Ile Asn Ala Arg Arg Asn Arg Ile Thr
       450                 455                 460
   Leu Thr Leu Asp Asp Glu Ala Ala Pro Pro Ala Pro Asp Ser Thr Trp
   465                 470                 475                 480
   Val Gln Ile Tyr Ser Gly Asn Ser Tyr Tyr Phe Gly Gly Cys Pro Asp
                   485                 490                 495
   Asn Leu Thr Asp Ser Gln Cys Leu Asn Pro Ile Lys Ala Phe Gln Gly
               500                 505                 510
   Cys Met Arg Leu Ile Phe Ile Asp Asn Gln Pro Lys Asp Leu Ile Ser
           515                 520                 525
   Val Gln Gln Gly Ser Leu Gly Asn Phe Ser Asp Leu His Ile Asp Leu
       530                 535                 540
   Cys Ser Ile Lys Asp Arg Cys Leu Pro Asn Tyr Cys Glu His Gly Gly
   545                 550                 555                 560
   Ser Cys Ser Gln Ser Trp Thr Thr Phe Tyr Cys Asn Cys Ser Asp Thr
                   565                 570                 575
   Ser Tyr Thr Gly Ala Thr Cys His Asn Ser Ile Tyr Glu Gln Ser Cys
               580                 585                 590
   Glu Val Tyr Arg His Gln Gly Asn Thr Ala Gly Phe Phe Tyr Ile Asp
           595                 600                 605
   Ser Asp Gly Ser Gly Pro Leu Gly Pro Leu Gln Val Tyr Cys Asn Ile
       610                 615                 620
   Thr Glu Asp Lys Ile Trp Thr Ser Val Gln His Asn Asn Thr Glu Leu
   625                 630                 635                 640
   Thr Arg Val Arg Gly Ala Asn Pro Glu Lys Pro Tyr Ala Met Ala Leu
                   645                 650                 655
   Asp Tyr Gly Gly Ser Met Glu Gln Leu Glu Ala Val Ile Asp Gly Ser
               660                 665                 670
   Glu His Cys Glu Gln Glu Val Ala Tyr His Cys Arg Arg Ser Arg Leu
           675                 680                 685
   Leu Asn Thr Pro Asp Gly Thr Pro Phe Thr Trp Trp Ile Gly Arg Ser
       690                 695                 700
   Asn Glu Arg His Pro Tyr Trp Gly Gly Ser Pro Pro Gly Val Gln Gln
   705                 710                 715                 720
   Cys Glu Cys Gly Leu Asp Glu Ser Cys Leu Asp Ile Gln His Phe Cys
                   725                 730                 735
   Asn Cys Asp Ala Asp Lys Asp Glu Trp Thr Asn Asp Thr Gly Phe Leu
               740                 745                 750
   Ser Phe Lys Asp His Leu Pro Val Thr Gln Ile Val Ile Thr Asp Thr
           755                 760                 765
   Asp Arg Ser Asn Ser Glu Ala Ala Trp Arg Ile Gly Pro Leu Arg Cys
       770                 775                 780
   Tyr Gly Asp Arg Glu Tyr Lys Ile Glu Arg Ser Phe Leu Ser Ala Leu
   785                 790                 795                 800
   His Glu His Lys Met Phe Leu Leu Pro Tyr Pro Phe Ser Leu Gln Cys
                   805                 810                 815
   Ala Leu Val Leu Lys Ile Ile His Met Ser Ser Ala Phe Pro Tyr Pro
               820                 825                 830
   Thr Glu Asn Asp Lys Pro Cys
           835
<210> 21
   <211> 2376
   <212> DNA
   <213> homo sapiens
<400> 21
   atggattctt taccacggct gaccagcgtt ttgactttgc tgttctctgg cttgtggcat 60
   ttaggattaa cagcgacaaa ctacaactgt gatgatccac tagcatccct gctctctcca 120
   atggcttttt ccagttcctc agacctcact ggcactcaca gcccagctca actcaactgg 180
   agagttggaa ctggcggttg gtccccagca gattccaatg ctcaacagtg gctccagatg 240
   gacctgggaa acagagtaga gattacagca gtggccacgc agggaagata cggaagctct 300
   gactgggtga cgagttacag cctgatgttc agtgacacag gacgcaactg gaaacagtac 360
   aaacaagaag acagcatctg gacctttgca ggaaacatga atgctgacag cgtggtgcac 420
   cacaagctat tgcactcagt gagagcccga tttgttcgct ttgtgcccct ggaatggaat 480
   cccagtggga agattggcat gagagtcgag gtctacggat gttcctataa atcagacgtt 540
   gctgactttg atggccgaag ctcacttctg tacaggttca atcagaagtt gatgagtact 600
   ctcaaagatg tgatctccct gaagttcaag agcatgcaag gagatggggt cctgttccat 660
   ggagaaggtc agcgtggaga ccacatcacc ttggaactcc agaaggggag gctcgcccta 720
   cacctcaatt tgggtgacag caaagcgcgg ctcagcagca gcttgccctc tgccaccctg 780
   ggcagcctcc tggatgacca gcactggcac tyggtcctca ttgagcgggt gggcaagcag 840
   gtgaacttca cggtggacaa gcacacacag cacttccgca ccaagggcga gacggatgcc 900
   ctagacattg actatgaggg caatgtcact ttttcctgct ccgaaccaca gattgtgccc 960
   atcacatttg tyaactccag cggcagctat ttgctgctgc ccggcacccc ccaaattgat 1020
   gggctctcag tgagtttcca gtttcgaaca tggaacaagg atggtctgct tctgtccaca 1080
   gagctgtctg agggctcggg aaccctgctg ctgagcctgg agggtggaat cctgagactc 1140
   gtgattcaga aaatgacaga acgcgtagct gaaatcctca caggcagcaa cttgaatgat 1200
   ggcctgtggc actcggttag catcaacgcc aggaggaacc gcatcacgct cactctggat 1260
   gatgaagcag cacccccggc tccagacagc acttgggtgc agatttattc tggaaatagc 1320
   tactattttg gagggtgccc cgacaatctc accgattccc aatgtttaaa tcccattaag 1380
   gctttccaag gctgcatgag gctcatcttt attgataacc agcccaagga cctcatttca 1440
   gttcagcaag gttccctggg gaattttagt gatttacaca ttgatctgtg tagcatcaaa 1500
   gacaggtgtt tgccaaacta ctgtgaacat ggaggaagct gctcccagtc ctggactacc 1560
   ttctattgta actgcagtga cacaagttac actggtgcca cctgccacaa ctccatctac 1620
   gagcaatcct gcgaggtgta caggcaccag gggaatacag ccggcttctt ctacatcgac 1680
   tcagatggca gcggcccact gggacctctc caggtgtact gcaatatcac tgaggacaag 1740
   atctggacat cagtgcagca caacaataca gagctgaccc gagtgcgggg cgctaaccct 1800
   gagaagccct atgccatggc cttggactac gggggcagca tggaacagct ggaggccgtg 1860
   atcgacggct ctgagcactg tgagcaggag gtggcctacc actgcaggag gtcccgcctg 1920
   ctcaacacgc cggatggaac accatttacc tggtggattg ggcggtccaa tgaaaggcac 1980
   ccttactggg gaggttcccc tcctggggtc cagcagtgtg agtgtggcct agacgagagc 2040
   tgcctggaca ttcagcactt ttgcaattgc gacgctgaca aggatgaatg gacaaatgat 2100
   actggctttc tttccttcaa agaccacttg cctgtcactc agatagttat cactgatacc 2160
   gacagatcaa actcagaagc cgcttggaga attggtccct tgcgttgcta tggtgaccgt 2220
   gagtacaaaa tcgaaagaag ctttctctct gcattacatg agcacaagat gttcttactc 2280
   ccttatccct tttccctgca gtgtgcccta gtcttgaaaa ttatccacat gtccagtgct 2340
   ttcccatacc ccactgaaaa cgataaacca tgttga 2376
<210> 22
   <211> 791
   <212> PRT
   <213> homo sapiens
<220>
   <221> VARIANT
   <222> (1)...(791)
   <223> Xaa = Any Amino Acid
<400> 22
   Met Asp Ser Leu Pro Arg Leu Thr Ser Val Leu Thr Leu Leu Phe Ser
    1               5                  10                  15
   Gly Leu Trp His Leu Gly Leu Thr Ala Thr Asn Tyr Asn Cys Asp Asp
               20                  25                  30
   Pro Leu Ala Ser Leu Leu Ser Pro Met Ala Phe Ser Ser Ser Ser Asp
           35                  40                  45
   Leu Thr Gly Thr His Ser Pro Ala Gln Leu Asn Trp Arg Val Gly Thr
       50                  55                  60
   Gly Gly Trp Ser Pro Ala Asp Ser Asn Ala Gln Gln Trp Leu Gln Met
   65                  70                  75                  80
   Asp Leu Gly Asn Arg Val Glu Ile Thr Ala Val Ala Thr Gln Gly Arg
                   85                  90                  95
   Tyr Gly Ser Ser Asp Trp Val Thr Ser Tyr Ser Leu Met Phe Ser Asp
               100                 105                 110
   Thr Gly Arg Asn Trp Lys Gln Tyr Lys Gln Glu Asp Ser Ile Trp Thr
           115                 120                 125
   Phe Ala Gly Asn Met Asn Ala Asp Ser Val Val His His Lys Leu Leu
       130                 135                 140
   His Ser Val Arg Ala Arg Phe Val Arg Phe Val Pro Leu Glu Trp Asn
   145                 150                 155                 160
   Pro Ser Gly Lys Ile Gly Met Arg Val Glu Val Tyr Gly Cys Ser Tyr
                   165                 170                 175
   Lys Ser Asp Val Ala Asp Phe Asp Gly Arg Ser Ser Leu Leu Tyr Arg
               180                 185                 190
   Phe Asn Gln Lys Leu Met Ser Thr Leu Lys Asp Val Ile Ser Leu Lys
           195                 200                 205
   Phe Lys Ser Met Gln Gly Asp Gly Val Leu Phe His Gly Glu Gly Gln
       210                 215                 220
   Arg Gly Asp His Ile Thr Leu Glu Leu Gln Lys Gly Arg Leu Ala Leu
   225                 230                 235                 240
   His Leu Asn Leu Gly Asp Ser Lys Ala Arg Leu Ser Ser Ser Leu Pro
                   245                 250                 255
   Ser Ala Thr Leu Gly Ser Leu Leu Asp Asp Gln His Trp His Xaa Val
               260                 265                 270
   Leu Ile Glu Arg Val Gly Lys Gln Val Asn Phe Thr Val Asp Lys His
           275                 280                 285
   Thr Gln His Phe Arg Thr Lys Gly Glu Thr Asp Ala Leu Asp Ile Asp
       290                 295                 300
   Tyr Glu Gly Asn Val Thr Phe Ser Cys Ser Glu Pro Gln Ile Val Pro
   305                 310                 315                 320
   Ile Thr Phe Val Asn Ser Ser Gly Ser Tyr Leu Leu Leu Pro Gly Thr
                   325                 330                 335
   Pro Gln Ile Asp Gly Leu Ser Val Ser Phe Gln Phe Arg Thr Trp Asn
               340                 345                 350
   Lys Asp Gly Leu Leu Leu Ser Thr Glu Leu Ser Glu Gly Ser Gly Thr
           355                 360                 365
   Leu Leu Leu Ser Leu Glu Gly Gly Ile Leu Arg Leu Val Ile Gln Lys
       370                 375                 380
   Met Thr Glu Arg Val Ala Glu Ile Leu Thr Gly Ser Asn Leu Asn Asp
   385                 390                 395                 400
   Gly Leu Trp His Ser Val Ser Ile Asn Ala Arg Arg Asn Arg Ile Thr
                   405                 410                 415
   Leu Thr Leu Asp Asp Glu Ala Ala Pro Pro Ala Pro Asp Ser Thr Trp
               420                 425                 430
   Val Gln Ile Tyr Ser Gly Asn Ser Tyr Tyr Phe Gly Gly Cys Pro Asp
           435                 440                 445
   Asn Leu Thr Asp Ser Gln Cys Leu Asn Pro Ile Lys Ala Phe Gln Gly
       450                 455                 460
   Cys Met Arg Leu Ile Phe Ile Asp Asn Gln Pro Lys Asp Leu Ile Ser
   465                 470                 475                 480
   Val Gln Gln Gly Ser Leu Gly Asn Phe Ser Asp Leu His Ile Asp Leu
                   485                 490                 495
   Cys Ser Ile Lys Asp Arg Cys Leu Pro Asn Tyr Cys Glu His Gly Gly
               500                 505                 510
   Ser Cys Ser Gln Ser Trp Thr Thr Phe Tyr Cys Asn Cys Ser Asp Thr
           515                 520                 525
   Ser Tyr Thr Gly Ala Thr Cys His Asn Ser Ile Tyr Glu Gln Ser Cys
       530                 535                 540
   Glu Val Tyr Arg His Gln Gly Asn Thr Ala Gly Phe Phe Tyr Ile Asp
   545                 550                 555                 560
   Ser Asp Gly Ser Gly Pro Leu Gly Pro Leu Gln Val Tyr Cys Asn Ile
                   565                 570                 575
   Thr Glu Asp Lys Ile Trp Thr Ser Val Gln His Asn Asn Thr Glu Leu
               580                 585                 590
   Thr Arg Val Arg Gly Ala Asn Pro Glu Lys Pro Tyr Ala Met Ala Leu
           595                 600                 605
   Asp Tyr Gly Gly Ser Met Glu Gln Leu Glu Ala Val Ile Asp Gly Ser
       610                 615                 620
   Glu His Cys Glu Gln Glu Val Ala Tyr His Cys Arg Arg Ser Arg Leu
   625                 630                 635                 640
   Leu Asn Thr Pro Asp Gly Thr Pro Phe Thr Trp Trp Ile Gly Arg Ser
                   645                 650                 655
   Asn Glu Arg His Pro Tyr Trp Gly Gly Ser Pro Pro Gly Val Gln Gln
               660                 665                 670
   Cys Glu Cys Gly Leu Asp Glu Ser Cys Leu Asp Ile Gln His Phe Cys
           675                 680                 685
   Asn Cys Asp Ala Asp Lys Asp Glu Trp Thr Asn Asp Thr Gly Phe Leu
       690                 695                 700
   Ser Phe Lys Asp His Leu Pro Val Thr Gln Ile Val Ile Thr Asp Thr
   705                 710                 715                 720
   Asp Arg Ser Asn Ser Glu Ala Ala Trp Arg Ile Gly Pro Leu Arg Cys
                   725                 730                 735
   Tyr Gly Asp Arg Glu Tyr Lys Ile Glu Arg Ser Phe Leu Ser Ala Leu
               740                 745                 750
   His Glu His Lys Met Phe Leu Leu Pro Tyr Pro Phe Ser Leu Gln Cys
           755                 760                 765
   Ala Leu Val Leu Lys Ile Ile His Met Ser Ser Ala Phe Pro Tyr Pro
       770                 775                 780
   Thr Glu Asn Asp Lys Pro Cys
   785 790
<210> 23
   <211> 3897
   <212> DNA
   <213> Homo sapiens
<400> 23
   atgtttcttg ctaatagaat ctggtctctc tccctctgtc ctcctatcat tatagacaac    60
   tgtgatgatc cactagcatc cctgctctct ccaatggctt tttccagttc ctcagacctc   120
   actggcactc acagcccagc tcaactcaac tggagagttg gaactggcgg ttggtcccca   180
   gcagattcca atgctcaaca gtggctccag atggacctgg gaaacagagt agagattaca   240
   gcagtggcca cgcagggaag atacggaagc tctgactggg tgacgagtta cagcctgatg   300
   ttcagtgaca caggacgcaa ctggaaacag tacaaacaag aagacagcat ctggaccttt   360
   gcaggaaaca tgaatgctga cagcgtggtg caccacaagc tattgcactc agtgagagcc   420
   cgatttgttc gctttgtgcc cctggaatgg aatcccagtg ggaagattgg catgagagtc   480
   gaggtctacg gatgttccta taaatcagac gttgctgact ttgatggccg aagctcactt   540
   ctgtacaggt tcaatcagaa gttgatgagt actctcaaag atgtgatctc cctgaagttc   600
   aagagcatgc aaggagatgg ggtcctgttc catggagaag gtcagcgtgg agaccacatc   660
   accttggaac tccagaaggg gaggctcgcc ctacacctca atttgggtga cagcaaagcg   720
   cggctcagca gcagcttgcc ctctgccacc ctgggcagcc tcctggatga ccagcactgg   780
   cactcggtcc tcattgagcg ggtgggcaag caggtgaact tcacggtgga caagcacaca   840
   cagcacttcc gcaccaaggg cgagacggat gccttagaca ttgactatga gcttagtttt   900
   ggaggaattc cagtaccagg aaaacctggg acctttttaa agaaaaactt ccatggatgc   960
   atcgaaaacc tttactacaa tggagtaaac ataattgacc tggctaagag acgaaagcat  1020
   cagatctata ctgtgggcaa tgtcactttt tcctgctccg aaccacagat tgtgcccatc  1080
   acatttgtca actccagcgg cagctatttg ctgctgcccg gcacccccca aattgatggg  1140
   ctctcagtga gtttccagtt tcgaacatgg aacaaggatg gtctgcttct gtccacagag  1200
   ctgtctgagg gctcgggaac cctgctgctg agcctggagg gtggaatcct gagactcgtg  1260
   attcagaaaa tgacagaacg cgtagctgaa atcctcacag gcagcaactt gaatgatggc  1320
   ctgtggcact cggttagcat caacgccagg aggaaccgca tcacgctcac tctggatgat  1380
   gaagcagcac ccccggctcc agacagcact tgggtgcaga tttattctgg aaatagctac  1440
   tattttggag ggtgccccga caatctcacc gattcccaat gtttaaatcc cattaaggct  1500
   ttccaaggct gcatgaggct catctttatt gataaccagc ccaaggacct catttcagtt  1560
   cagcaaggtt ccctggggaa ttttagtgat ttacacattg atctgtgtag catcaaagac  1620
   aggtgtttgc caaactactg tgaacatgga ggaagctgct cccagtcctg gactaccttc  1680
   tattgtaact gcagtgacac aagttacact ggtgccacct gccacaactc catctacgag  1740
   caatcctgcg aggtgtacag gcaccagggg aatacagccg gcttcttcta catcgactca  1800
   gatggcagcg gcccactggg acctctccag gtgtactgca atatcactga ggacaagatc  1860
   tggacatcag tgcagcacaa caatacagag ctgacccgag tgcggggcgc taaccctgag  1920
   aagccctatg ccatggcctt ggactacggg ggcagcatgg aacagctgga ggccgtgatc  1980
   gacggctctg agcactgtga gcaggaggtg gcctaccact gcaggaggtc ccgcctgctc  2040
   aacacgccgg atggaacacc atttacctgg tggattgggc ggtccaatga aaggcaccct  2100
   tactggggag gttcccctcc tggggtccag cagtgtgagt gtggcctaga cgagagctgc  2160
   ctggacattc agcacttttg caattgcgac gctgacaagg atgaatggac aaatgatact  2220
   ggctttcttt ccttcaaaga ccacttgcct gtcactcaga tagttatcac tgataccgac  2280
   agatcaaact cagaagccgc ttggagaatt ggtcccttgc gttgctatgg tgaccgacgc  2340
   ttctggaacg ccgtctcatt ttatacagaa gcctcttacc tccactttcc taccttccat  2400
   gcggaattca gtgccgatat ttccttcttt tttaaaacca cagcattatc cggagttttc  2460
   ctagaaaatc ttggcattaa agacttcatt cgactcgaaa taagctctcc ttcagagatc  2520
   acctttgcca tcgatgttgg gaatggtcct gtggagcttg tagtccagtc tccttctctt  2580
   ctgaatgaca accaatggca ctatgtccgg gctgagagga acctcaagga gacctccctg  2640
   caggtggaca accttccaag gagcaccagg gagacgtcgg aggagggcca ttttcgactg  2700
   cagctgaaca gccagttgtt tgtaggggga acgtcatcca gacagaaagg cttcctagga  2760
   tgcattcgct ccttacactt gaatggacag aaaatggacc tggaagagag ggcaaaggtc  2820
   acatctggag tcaggccagg ctgccccggc cactgcagca gctacggcag catctgccac    2880
   aacgggggca agtgtgtgga gaagcacaat ggctacctgt gtgattgcac caattcacct    2940
   tatgaagggc ccttttgcaa aaaagaggtt tctgctgttt ttgaggctgg cacgtcggtt    3000
   acttacatgt ttcaagaacc ctatcctgtg accaagaata taagcctctc atcctcagct    3060
   atttacacag attcagctcc atccaaggaa aacattgcac ttagctttgt gacaacccag    3120
   gcacccagtc ttttgctctt tatcaattct tcttctcagg acttcgtggt tgttctgctc    3180
   tgcaagaatg gaagcttaca ggttcgctat cacctaaaca aggaagaaac ccatgtattc    3240
   accattgatg cagataactt tgctaacaga aggatgcacc acttgaagat taaccgagag    3300
   ggaagagagc ttaccattca gatggaccag caacttcgac tcagttataa cttctctccg    3360
   gaagtagagt tcagggttat aaggtcactc accttgggca aagtcacaga gaatcttggt    3420
   ttggattctg aagttgctaa agcaaatgcc atgggttttg ctggatgcat gtcttccgtc    3480
   cagtacaacc acatagcacc actgaaggct gccctgcgcc atgccactgt cgcgcctgtg    3540
   actgtccatg ggaccttgac ggaatccagc tgtggcttca tggtggactc agatgtgaat    3600
   gcagtgacca cggtgcattc ttcatcagat ccttttggga agacagatga gcgggaacca    3660
   ctcacaaatg ctgttcgaag tgattcggca gtcatcggag gggtgatagc agtggtgata     3720
   ttcatcatct tctgtatcat cggcatcatg acccggttcc tctaccagca caagcagtca    3780
   catcgtacga gccagatgaa ggagaaggaa tatccagaaa atttggacag ttccttcaga    3840
   aatgaaattg acttgcaaaa cacagtgagc gagtgtaaac gggaatattt catctga       3897
<210> 24
   <211> 1298
   <222> PRT
   <213> Homo sapiens
<400> 24
   Met Phe Leu Ala Asn Arg Ile Trp Ser Leu Ser Leu Cys Pro Pro Ile
   1               5                   10                  15
   Ile Ile Asp Asn Cys Asp Asp Pro Leu Ala Ser Leu Leu Ser Pro Met
               20                  25                  30
   Ala Phe Ser Ser Ser Ser Asp Leu Thr Gly Thr His Ser Pro Ala Gln
           35                  40                  45
   Leu Asn Trp Arg Val Gly Thr Gly Gly Trp Ser Pro Ala Asp Ser Asn
       50                  55                  60
   Ala Gln Gln Trp Leu Gln Met Asp Leu Gly Asn Arg Val Glu Ile Thr
   65                  70                  75                  80
   Ala Val Ala Thr Gln Gly Arg Tyr Gly Ser Ser Asp Trp Val Thr Ser
                   85                  90                  95
   Tyr Ser Leu Met Phe Ser Asp Thr Gly Arg Asn Trp Lys Gln Tyr Lys
               100                 105                 110
   Gln Glu Asp Ser Ile Trp Thr Phe Ala Gly Asn Met Asn Ala Asp Ser
           115                 120                 125
   Val Val His His Lys Leu Leu His Ser Val Arg Ala Arg Phe Val Arg
       130                 135                 140
   Phe Val Pro Leu Glu Trp Asn Pro Ser Gly Lys Ile Gly Met Arg Val
   145                 150                 155                 160
   Glu Val Tyr Gly Cys Ser Tyr Lys Ser Asp Val Ala Asp Phe Asp Gly
                   165                 170                 175
   Arg Ser Ser Leu Leu Tyr Arg Phe Asn Gln Lys Leu Met Ser Thr Leu
               180                 185                 190
   Lys Asp Val Ile Ser Leu Lys Phe Lys Ser Met Gln Gly Asp Gly Val
           195                 200                 205
   Leu Phe His Gly Glu Gly Gln Arg Gly Asp His Ile Thr Leu Glu Leu
       210                 215                 220
   Gln Lys Gly Arg Leu Ala Leu His Leu Asn Leu Gly Asp Ser Lys Ala
   225                 230                 235                 240
   Arg Leu Ser Ser Ser Leu Pro Ser Ala Thr Leu Gly Ser Leu Leu Asp
                   245                 250                 255
   Asp Gln His Trp His Ser Val Leu Ile Glu Arg Val Gly Lys Gln Val
               260                 265                 270
   Asn Phe Thr Val Asp Lys His Thr Gln His Phe Arg Thr Lys Gly Glu
           275                 280                 285
   Thr Asp Ala Leu Asp Ile Asp Tyr Glu Leu Ser Phe Gly Gly Ile Pro
       290                 295                 300
   Val Pro Gly Lys Pro Gly Thr Phe Leu Lys Lys Asn Phe His Gly Cys
   305                 310                 315                 320
   Ile Glu Asn Leu Tyr Tyr Asn Gly Val Asn Ile Ile Asp Leu Ala Lys
                   325                 330                 335
   Arg Arg Lys His Gln Ile Tyr Thr Val Gly Asn Val Thr Phe Ser Cys
               340                 345                 350
   Ser Glu Pro Gln Ile Val Pro Ile Thr Phe Val Asn Ser Ser Gly Ser
           355                 360                 365
   Tyr Leu Leu Leu Pro Gly Thr Pro Gln Ile Asp Gly Leu Ser Val Ser
       370                 375                 380
   Phe Gln Phe Arg Thr Trp Asn Lys Asp Gly Leu Leu Leu Ser Thr Glu
   385                 390                 395                 400
   Leu Ser Glu Gly Ser Gly Thr Leu Leu Leu Ser Leu Glu Gly Gly Ile
                   405                 410                 415
   Leu Arg Leu Val Ile Gln Lys Met Thr Glu Arg Val Ala Glu Ile Leu
               420                 425                 430
   Thr Gly Ser Asn Leu Asn Asp Gly Leu Trp His Ser Val Ser Ile Asn
           435                 440                 445
   Ala Arg Arg Asn Arg Ile Thr Leu Thr Leu Asp Asp Glu Ala Ala Pro
       450                 455                 460
   Pro Ala Pro Asp Ser Thr Trp Val Gln Ile Tyr Ser Gly Asn Ser Tyr
   465                 470                 475                 480
   Tyr Phe Gly Gly Cys Pro Asp Asn Leu Thr Asp Ser Gln Cys Leu Asn
                   485                 490                 495
   Pro Ile Lys Ala Phe Gln Gly Cys Met Arg Leu Ile Phe Ile Asp Asn
               500                 505                 510
   Gln Pro Lys Asp Leu Ile Ser Val Gln Gln Gly Ser Leu Gly Asn Phe
           515                 520                 525
   Ser Asp Leu His Ile Asp Leu Cys Ser Ile Lys Asp Arg Cys Leu Pro
       530                 535                 540
   Asn Tyr Cys Glu His Gly Gly Ser Cys Ser Gln Ser Trp Thr Thr Phe
   545                 550                 555                 560
   Tyr Cys Asn Cys Ser Asp Thr Ser Tyr Thr Gly Ala Thr Cys His Asn
                   565                 570                 575
   Ser Ile Tyr Glu Gln Ser Cys Glu Val Tyr Arg His Gln Gly Asn Thr
               580                 585                 590
   Ala Gly Phe Phe Tyr Ile Asp Ser Asp Gly Ser Gly Pro Leu Gly Pro
           595                 600                 605
   Leu Gln Val Tyr Cys Asn Ile Thr Glu Asp Lys Ile Trp Thr Ser Val
       610                 615                 620
   Gln His Asn Asn Thr Glu Leu Thr Arg Val Arg Gly Ala Asn Pro Glu
   625                 630                 635                 640
   Lys Pro Tyr Ala Met Ala Leu Asp Tyr Gly Gly Ser Met Glu Gln Leu
                   645                 650                 655
   Glu Ala Val Ile Asp Gly Ser Glu His Cys Glu Gln Glu Val Ala Tyr
               660                 665                 670
   His Cys Arg Arg Ser Arg Leu Leu Asn Thr Pro Asp Gly Thr Pro Phe
           675                 680                 685
   Thr Trp Trp Ile Gly Arg Ser Asn Glu Arg His Pro Tyr Trp Gly Gly
       690                 695                 700
   Ser Pro Pro Gly Val Gln Gln Cys Glu Cys Gly Leu Asp Glu Ser Cys
   705                 710                 715                 720
   Leu Asp Ile Gln His Phe Cys Asn Cys Asp Ala Asp Lys Asp Glu Trp
                   725                 730                 735
   Thr Asn Asp Thr Gly Phe Leu Ser Phe Lys Asp His Leu Pro Val Thr
               740                 745                 750
   Gln Ile Val Ile Thr Asp Thr Asp Arg Ser Asn Ser Glu Ala Ala Trp
           755                 760                 765
   Arg Ile Gly Pro Leu Arg Cys Tyr Gly Asp Arg Arg Phe Trp Asn Ala
       770                 775                 780
   Val Ser Phe Tyr Thr Glu Ala Ser Tyr Leu His Phe Pro Thr Phe His
   785                 790                 795                 800
   Ala Glu Phe Ser Ala Asp Ile Ser Phe Phe Phe Lys Thr Thr Ala Leu
                   805                 810                 815
   Ser Gly Val Phe Leu Glu Asn Leu Gly Ile Lys Asp Phe Ile Arg Leu
               820                 825                 830
   Glu Ile Ser Ser Pro Ser Glu Ile Thr Phe Ala Ile Asp Val Gly Asn
           835                 840                 845
   Gly Pro Val Glu Leu Val Val Gln Ser Pro Ser Leu Leu Asn Asp Asn
       850                 855                 860
   Gln Trp His Tyr Val Arg Ala Glu Arg Asn Leu Lys Glu Thr Ser Leu
   865                 870                 875                 880
   Gln Val Asp Asn Leu Pro Arg Ser Thr Arg Glu Thr Ser Glu Glu Gly
                   885                 890                 895
   His Phe Arg Leu Gln Leu Asn Ser Gln Leu Phe Val Gly Gly Thr Ser
               900                 905                 910
   Ser Arg Gln Lys Gly Phe Leu Gly Cys Ile Arg Ser Leu His Leu Asn
           915                 920                 925
   Gly Gln Lys Met Asp Leu Glu Glu Arg Ala Lys Val Thr Ser Gly Val
       930                 935                 940
   Arg Pro Gly Cys Pro Gly His Cys Ser Ser Tyr Gly Ser Ile Cys His
   945                 950                 955                 960
   Asn Gly Gly Lys Cys Val Glu Lys His Asn Gly Tyr Leu Cys Asp Cys
                   965                 970                 975
   Thr Asn Ser Pro Tyr Glu Gly Pro Phe Cys Lys Lys Glu Val Ser Ala
               980                 985                 990
   Val Phe Glu Ala Gly Thr Ser Val Thr Tyr Met Phe Gln Glu Pro Tyr
           995                 1000                1005
   Pro Val Thr Lys Asn Ile Ser Leu Ser Ser Ser Ala Ile Tyr Thr Asp
       1010                1015                1020
   Ser Ala Pro Ser Lys Glu Asn Ile Ala Leu Ser Phe Val Thr Thr Gln
   1025                1030                1035                1040
   Ala Pro Ser Leu Leu Leu Phe Ile Asn Ser Ser Ser Gln Asp Phe Val
                   1045                1050                1055
   Val Val Leu Leu Cys Lys Asn Gly Ser Leu Gln Val Arg Tyr His Leu
               1060                1065                1070
   Asn Lys Glu Glu Thr His Val Phe Thr Ile Asp Ala Asp Asn Phe Ala
           1075                1080                1085
   Asn Arg Arg Met His His Leu Lys Ile Asn Arg Glu Gly Arg Glu Leu
       1090                1095                1100
   Thr Ile Gln Met Asp Gln Gln Leu Arg Leu Ser Tyr Asn Phe Ser Pro
   1105                1110                1115                1120
   Glu Val Glu Phe Arg Val Ile Arg Ser Leu Thr Leu Gly Lys Val Thr
                   1125                1130                1135
   Glu Asn Leu Gly Leu Asp Ser Glu Val Ala Lys Ala Asn Ala Met Gly
                   1140                    1145                    1150
   Phe Ala Gly Cys Met Ser Ser Val Gln Tyr Asn His Ile Ala Pro Leu
               1155                    1160                    1165
   Lys Ala Ala Leu Arg His Ala Thr Val Ala Pro Val Thr Val His Gly
           1170                    1175                    1180
   Thr Leu Thr Glu Ser Ser Cys Gly Phe Met Val Asp Ser Asp Val Asn
   1185                    1190                    1195                    1200
   Ala Val Thr Thr Val His Ser Ser Ser Asp Pro Phe Gly Lys Thr Asp
                       1205                    1210                    1215
   Glu Arg Glu Pro Leu Thr Asn Ala Val Arg Ser Asp Ser Ala Val Ile
                   1220                    1225                    1230
   Gly Gly Val Ile Ala Val Val Ile Phe Ile Ile Phe Cys Ile Ile Gly
               1235                    1240                    1245
   Ile Met Thr Arg Phe Leu Tyr Gln His Lys Gln Ser His Arg Thr Ser
           1250                    1255                    1260
   Gln Met Lys Glu Lys Glu Tyr Pro Glu Asn Leu Asp Ser Ser Phe Arg
   1265                    1270                    1275                    1280
   Asn Glu Ile Asp Leu Gln Asn Thr Val Ser Glu Cys Lys Arg Glu Tyr
                       1285                    1290                    1295
   Phe Ile
<210> 25
   <211> 3528
   <212> DNA
   <213> Homo sapiens
<400> 25
   atgaatgctg acagcgtggt gcaccacaag ctattgcact cagtgagagc ccgatttgtt 60
   cgctttgtgc ccctggaatg gaatcccagt gggaagattg gcatgagagt cgaggtctac 120
   ggatgttcct ataaatcaga cgttgctgac tttgatggcc gaagctcact tctgtacagg 180
   ttcaatcaga agttgatgag tactctcaaa gatgtgatct ccctgaagtt caagagcatg 240
   caaggagatg gggtcctgtt ccatggagaa ggtcagcgtg gagaccacat caccttggaa 300
   ctccagaagg ggaggctcgc cctacacctc aatttgggtg acagcaaagc gcggctcagc 360
   agcagcttgc cctctgccac cctgggcagc ctcctggatg accagcactg gcactcggtc 420
   ctcattgagc gggtgggcaa gcaggtgaac ttcacggtgg acaagcacac acagcacttc 480
   cgcaccaagg gcgagacgga tgccttagac attgactatg agcttagttt tggaggaatt 540
   ccagtaccag gaaaacctgg gaccttttta aagaaaaact tccatggatg catcgaaaac 600
   ctttactaca atggagtaaa cataattgac ctggctaaga gacgaaagca tcagatctat 660
   actgtgggca atgtcacttt ttcctgctcc gaaccacaga ttgtgcccat cacatttgtc 720
   aactccagcg gcagctattt gctgctgccc ggcacccccc aaattgatgg gctctcagtg 780
   agtttccagt ttcgaacatg gaacaaggat ggtctgcttc tgtccacaga gctgtctgag 840
   ggctcgggaa ccctgctgct gagcctggag ggtggaatcc tgagactcgt gattcagaaa 900
   atgacagaac gcgtagctga aatcctcaca ggcagcaact tgaatgatgg cctgtggcac 960
   tcggttagca tcaacgccag gaggaaccgc atcacgctca ctctggatga tgaagcagca 1020
   cccccggctc cagacagcac ttgggtgcag atttattctg gaaatagcta ctattttgga 1080
   gggtgccccg acaatctcac cgattcccaa tgtttaaatc ccattaaggc tttccaaggc 1140
   tgcatgaggc tcatctttat tgataaccag cccaaggacc tcatttcagt tcagcaaggt 1200
   tccctgggga attttagtga tttacacatt gatctgtgta gcatcaaaga caggtgtttg 1260
   ccaaactact gtgaacatgg aggaagctgc tcccagtcct ggactacctt ctattgtaac 1320
   tgcagtgaca caagttacac tggtgccacc tgccacaact ccatctacga gcaatcctgc 1380
   gaggtgtaca ggcaccaggg gaatacagcc ggcttcttct acatcgactc agatggcagc 1440
   ggcccactgg gacctctcca ggtgtactgc aatatcactg aggacaagat ctggacatca 1500
   gtgcagcaca acaatacaga gctgacccga gtgcggggcg ctaaccctga gaagccctat 1560
   gccatggcct tggactacgg gggcagcatg gaacagctgg aggccgtgat cgacggctct 1620
   gagcactgtg agcaggaggt ggcctaccac tgcaggaggt cccgcctgct caacacgccg 1680
   gatggaacac catttacctg gtggattggg cggtccaatg aaaggcaccc ttactgggga     1740
   ggttcccctc ctggggtcca gcagtgtgag tgtggcctag acgagagctg cctggacatt     1800
   cagcactttt gcaattgcga cgctgacaag gatgaatgga caaatgatac tggctttctt     1860
   tccttcaaag accacttgcc tgtcactcag atagttatca ctgataccga cagatcaaac     1920
   tcagaagccg cttggagaat tggtcccttg cgttgctatg gtgaccgacg cttctggaac     1980
   gccgtctcat tttatacaga agcctcttac ctccactttc ctaccttcca tgcggaattc     2040
   agtgccgata tttccttctt ttttaaaacc acagcattat ccggagtttt cctagaaaat     2100
   cttggcatta aagacttcat tcgactcgaa ataagctctc cttcagagat cacctttgcc     2160
   atcgatgttg ggaatggtcc tgtggagctt gtagtccagt ctccttctct tctgaatgac     2220
   aaccaatggc actatgtccg ggctgagagg aacctcaagg agacctccct gcaggtggac     2280
   aaccttccaa ggagcaccag ggagacgtcg gaggagggcc attttcgact gcagctgaac     2340
   agccagttgt ttctaggggg aacgtcatcc agacagaaag gcttcctagg atgcattcgc     2400
   tccttacact tgaatggaca gaaaatggac ctggaagaga gggcaaaggt cacatctgga     2460
   gtcaggccag gctgccccgg ccactgcagc agctacggca gcatctgcca caacgggggc      2520
   aagtgtgtgg agaagcacaa tggctacctg tgtgattgca ccaattcacc ttatgaaggg     2580
   cccttttgca aaaaagaggt ttctgctgtt tttgaggctg gcacgtcggt tacttacatg    2640
   tttcaagaac cctatcctgt gaccaagaat ataagcctct catcctcagc tatttacaca     2700
   gattcagctc catccaagga aaacattgca cttagctttg tgacaaccca ggcacccagt     2760
   cttttgctct ttatcaattc ttcttctcag gacttcgtgg ttgttctgct ctgcaagaat     2820
   ggaagcttac aggttcgcta tcacctaaac aaggaagaaa cccatgtatt caccattgat     2880
   gcagataact ttgctaacag aaggatgcac cacttgaaga ttaaccgaga gggaagagag     2940
   cttaccattc agatggacca gcaacttcga ctcagttata acttctctcc ggaagtagag     3000
   ttcagggtta taaggtcact caccttgggc aaagtcacag agaatcttgg tttggattct     3060
   gaagttgcta aagcaaatgc catgggtttt gctggatgca tgtcttccgt ccagtacaac     3120
   cacatagcac cactgaaggc tgccctgcgc catgccactg tcgcgcctgt gactgtccat     3180
   gggaccttga cggaatccag ctgtggcttc atggtggact cagatgtgaa tgcagtgacc     3240
   acggtgcatt cttcatcaga tccttttggg aagacagatg agcgggaacc actcacaaat    3300
   gctgttcgaa gtgattcggc agtcatcgga ggggtgatag cagtggtgat attcatcatc     3360
   ttctgtatca tcggcatcat gacccggttc ctctaccagc acaagcagtc acatcgtacg     3420
   agccagatga aggagaagga atatccagaa aatttggaca gttccttcag aaatgaaatt     3480
   gacttgcaaa acacagtgag cgagtgtaaa cgggaatatt tcatctga                   3528
<210> 26
   <211> 1175
   <212> PRT
   <213> Homo sapiens
<400> 26
   Met Asn Ala Asp Ser Val Val His His Lys Leu Leu His Ser Val Arg
    1               5                  10                  15
   Ala Arg Phe Val Arg Phe Val Pro Leu Glu Trp Asn Pro Ser Gly Lys
               20                  25                  30
   Ile Gly Met Arg Val Glu Val Tyr Gly Cys Ser Tyr Lys Ser Asp Val
           35                  40                  45
   Ala Asp Phe Asp Gly Arg Ser Ser Leu Leu Tyr Arg Phe Asn Gln Lys
       50                  55                  60
   Leu Met Ser Thr Leu Lys Asp Val Ile Ser Leu Lys Phe Lys Ser Met
   65                  70                  75                  80
   Gln Gly Asp Gly Val Leu Phe His Gly Glu Gly Gln Arg Gly Asp His
                   85                  90                  95
   Ile Thr Leu Glu Leu Gln Lys Gly Arg Leu Ala Leu His Leu Asn Leu
               100                 105                 110
   Gly Asp Ser Lys Ala Arg Leu Ser Ser Ser Leu Pro Ser Ala Thr Leu
           115                 120                 125
   Gly Ser Leu Leu Asp Asp Gln His Trp His Ser Val Leu Ile Glu Arg
       130                 135                 140
   Val Gly Lys Gln Val Asn Phe Thr Val Asp Lys His Thr Gln His Phe
   145 150 155 160
   Arg Thr Lys Gly Glu Thr Asp Ala Leu Asp Ile Asp Tyr Glu Leu Ser
   165 170 175
   Phe Gly Gly Ile Pro Val Pro Gly Lys Pro Gly Thr Phe Leu Lys Lys
   180 185 190
   Asn Phe His Gly Cys Ile Glu Asn Leu Tyr Tyr Asn Gly Val Asn Ile
   195 200 205
   Ile Asp Leu Ala Lys Arg Arg Lys His Gln Ile Tyr Thr Val Gly Asn
   210 215 220
   Val Thr Phe Ser Cys Ser Glu Pro Gln Ile Val Pro Ile Thr Phe Val
   225 230 235 240
   Asn Ser Ser Gly Ser Tyr Leu Leu Leu Pro Gly Thr Pro Gln Ile Asp
   245 250 255
   Gly Leu Ser Val Ser Phe Gln Phe Arg Thr Trp Asn Lys Asp Gly Leu
   260 265 270
   Leu Leu Ser Thr Glu Leu Ser Glu Gly Ser Gly Thr Leu Leu Leu Ser
   275 280 285
   Leu Glu Gly Gly Ile Leu Arg Leu Val Ile Gln Lys Met Thr Glu Arg
   290 295 300
   Val Ala Glu Ile Leu Thr Gly Ser Asn Leu Asn Asp Gly Leu Trp His
   305 310 315 320
   Ser Val Ser Ile Asn Ala Arg Arg Asn Arg Ile Thr Leu Thr Leu Asp
   325 330 335
   Asp Glu Ala Ala Pro Pro Ala Pro Asp Ser Thr Trp Val Gln Ile Tyr
   340 345 350
   Ser Gly Asn Ser Tyr Tyr Phe Gly Gly Cys Pro Asp Asn Leu Thr Asp
   355 360 365
   Ser Gln Cys Leu Asn Pro Ile Lys Ala Phe Gln Gly Cys Met Arg Leu
   370 375 380
   Ile Phe Ile Asp Asn Gln Pro Lys Asp Leu Ile Ser Val Gln Gln Gly
   385 390 395 400
   Ser Leu Gly Asn Phe Ser Asp Leu His Ile Asp Leu Cys Ser Ile Lys
   405 410 415
   Asp Arg Cys Leu Pro Asn Tyr Cys Glu His Gly Gly Ser Cys Ser Gln
   420 425 430
   Ser Trp Thr Thr Phe Tyr Cys Asn Cys Ser Asp Thr Ser Tyr Thr Gly
   435 440 445
   Ala Thr Cys His Asn Ser Ile Tyr Glu Gln Ser Cys Glu Val Tyr Arg
   450 455 460
   His Gln Gly Asn Thr Ala Gly Phe Phe Tyr Ile Asp Ser Asp Gly Ser
   465 470 475 480
   Gly Pro Leu Gly Pro Leu Gln Val Tyr Cys Asn Ile Thr Glu Asp Lys
   485 490 495
   Ile Trp Thr Ser Val Gln His Asn Asn Thr Glu Leu Thr Arg Val Arg
   500 505 510
   Gly Ala Asn Pro Glu Lys Pro Tyr Ala Met Ala Leu Asp Tyr Gly Gly
   515 520 525
   Ser Met Glu Gln Leu Glu Ala Val Ile Asp Gly Ser Glu His Cys Glu
   530 535 540
   Gln Glu Val Ala Tyr His Cys Arg Arg Ser Arg Leu Leu Asn Thr Pro
   545 550 555 560
   Asp Gly Thr Pro Phe Thr Trp Trp Ile Gly Arg Ser Asn Glu Arg His
   565 570 575
   Pro Tyr Trp Gly Gly Ser Pro Pro Gly Val Gln Gln Cys Glu Cys Gly
   580 585 590
   Leu Asp Glu Ser Cys Leu Asp Ile Gln His Phe Cys Asn Cys Asp Ala
           595                 600                 605
   Asp Lys Asp Glu Trp Thr Asn Asp Thr Gly Phe Leu Ser Phe Lys Asp
       610                 615                 620
   His Leu Pro Val Thr Gln Ile Val Ile Thr Asp Thr Asp Arg Ser Asn
   625                 630                 635                 640
   Ser Glu Ala Ala Trp Arg Ile Gly Pro Leu Arg Cys Tyr Gly Asp Arg
                   645                 650                 655
   Arg Phe Trp Asn Ala Val Ser Phe Tyr Thr Glu Ala Ser Tyr Leu His
               660                 665                 670
   Phe Pro Thr Phe His Ala Glu Phe Ser Ala Asp Ile Ser Phe Phe Phe
           675                 680                 685
   Lys Thr Thr Ala Leu Ser Gly Val Phe Leu Glu Asn Leu Gly Ile Lys
       690                 695                 700
   Asp Phe Ile Arg Leu Glu Ile Ser Ser Pro Ser Glu Ile Thr Phe Ala
   705                 710                 715                 720
   Ile Asp Val Gly Asn Gly Pro Val Glu Leu Val Val Gln Ser Pro Ser
                   725                 730                 735
   Leu Leu Asn Asp Asn Gln Trp His Tyr Val Arg Ala Glu Arg Asn Leu
               740                 745                 750
   Lys Glu Thr Ser Leu Gln Val Asp Asn Leu Pro Arg Ser Thr Arg Glu
           755                 760                 765
   Thr Ser Glu Glu Gly His Phe Arg Leu Gln Leu Asn Ser Gln Leu Phe
       770                 775                 780
   Val Gly Gly Thr Ser Ser Arg Gln Lys Gly Phe Leu Gly Cys Ile Arg
   785                 790                 795                 800
   Ser Leu His Leu Asn Gly Gln Lys Met Asp Leu Glu Glu Arg Ala Lys
                   805                 810                 815
   Val Thr Ser Gly Val Arg Pro Gly Cys Pro Gly His Cys Ser Ser Tyr
               820                 825                 830
   Gly Ser Ile Cys His Asn Gly Gly Lys Cys Val Glu Lys His Asn Gly
           835                 840                 845
   Tyr Leu Cys Asp Cys Thr Asn Ser Pro Tyr Glu Gly Pro Phe Cys Lys
       850                 855                 860
   Lys Glu Val Ser Ala Val Phe Glu Ala Gly Thr Ser Val Thr Tyr Met
   865                 870                 875                 880
   Phe Gln Glu Pro Tyr Pro Val Thr Lys Asn Ile Ser Leu Ser Ser Ser
                   885                 890                 895
   Ala Ile Tyr Thr Asp Ser Ala Pro Ser Lys Glu Asn Ile Ala Leu Ser
               900                 905                 910
   Phe Val Thr Thr Gln Ala Pro Ser Leu Leu Leu Phe Ile Asn Ser Ser
           915                 920                 925
   Ser Gln Asp Phe Val Val Val Leu Leu Cys Lys Asn Gly Ser Leu Gln
       930                 935                 940
   Val Arg Tyr His Leu Asn Lys Glu Glu Thr His Val Phe Thr Ile Asp
   945                 950                 955                 960
   Ala Asp Asn Phe Ala Asn Arg Arg Met His His Leu Lys Ile Asn Arg
                   965                 970                 975
   Glu Gly Arg Glu Leu Thr Ile Gln Met Asp Gln Gln Leu Arg Leu Ser
               980                 985                 990
   Tyr Asn Phe Ser Pro Glu Val Glu Phe Arg Val Ile Arg Ser Leu Thr
           995                 1000                1005
   Leu Gly Lys Val Thr Glu Asn Leu Gly Leu Asp Ser Glu Val Ala Lys
       1010                1015                1020
   Ala Asn Ala Met Gly Phe Ala Gly Cys Met Ser Ser Val Gln Tyr Asn
   1025                1030                1035                1040
   His Ile Ala Pro Leu Lys Ala Ala Leu Arg His Ala Thr Val Ala Pro
   1045 1050 1055
   Val Thr Val His Gly Thr Leu Thr Glu Ser Ser Cys Gly Phe Met Val
   1060 1065 1070
   Asp Ser Asp Val Asn Ala Val Thr Thr Val His Ser Ser Ser Asp Pro
   1075 1080 1085
   Phe Gly Lys Thr Asp Glu Arg Glu Pro Leu Thr Asn Ala Val Arg Ser
   1090 1095 1100
   Asp Ser Ala Val Ile Gly Gly Val Ile Ala Val Val Ile Phe Ile Ile
   1105 1110 1115 1120
   Phe Cys Ile Ile Gly Ile Met Thr Arg Phe Leu Tyr Gln His Lys Gln
   1125 1130 1135
   Ser His Arg Thr Ser Gln Met Lys Glu Lys Glu Tyr Pro Glu Asn Leu
   1140 1145 1150
   Asp Ser Ser Phe Arg Asn Glu Ile Asp Leu Gln Asn Thr Val Ser Glu
   1155 1160 1165
   Cys Lys Arg Glu Tyr Phe Ile
   1170 1175
<210> 27
   <211> 4869
   <212> DNA
   <213> Homo sapiens
<400> 27
   attgggtttg gatttgcacc gttaaggagg ggggaagaga aggaagaggc gggcgaggaa 60
   ggcgagtcca gctagcggct gttgcgggga ccgtagcccc agctgcagct ccgaagaatc 120
   ccccgccacg gtttcggtgg agcgtctggg cacgggatgg agtgaaagag cgagtgcctc 180
   tccaagcggg ggtgggaggg ggtcaggctg tgcagaggag agagacagcg agaagaagcc 240
   gcggctggct actgcgaatt tgggattcga ttgggaggga ccgctcactc gggggaaatg 300
   gattctttac cacggctgac cagcgttttg actttgctgt tctctggctt gtggcattta 360
   ggattaacag cgacaaactg tgaggagtag atgcagtaac acatggaaac cgggagtacc 420
   tgaccttgtt cagagcaggt ggttaataaa tgatggtagt taatttctca acttctcttc 480
   ttatggaatg tttcttgcta atagaatctg gtctctctcc ctctgtcctc ctatcattat 540
   agacaactgt gatgatccac tagcatccct gctctctcca atggcttttt ccagttcctc 600
   agacctcact ggcactcaca gcccagctca actcaactgg agagttggaa ctggcggttg 660
   gtccccagca gattccaatg ctcaacagtg gctccagatg gacctgggaa acagagtaga 720
   gattacagca gtggccacgc agggaagata cggaagctct gactgggtga cgagttacag 780
   cctgatgttc agtgacacag gacgcaactg gaaacagtac aaacaagaag acagcatctg 840
   gacctttgca ggaaacatga atgctgacag cgtggtgcac cacaagctat tgcactcagt 900
   gagagcccga tttgttcgct ttgtgcccct ggaatggaat cccagtggga agattggcat 960
   gagagtcgag gtctacggat gttcctataa atcagacgtt gctgactttg atggccgaag 1020
   ctcacttctg tacaggttca atcagaagtt gatgagtact ctcaaagatg tgatctccct 1080
   gaagttcaag agcatgcaag gagatggggt cctgttccat ggagaaggtc agcgtggaga 1140
   ccacatcacc ttggaactcc agaaggggag gctcgcccta cacctcaatt tgggtgacag 1200
   caaagcgcgg ctcagcagca gcttgccctc tgccaccctg ggcagcctcc tggatgacca 1260
   gcactggcac tcggtcctca ttgagcgggt gggcaagcag gtgaacttca cggtggacaa 1320
   gcacacacag cacttccgca ccaagggcga gacggatgcc ttagacattg actatgagct 1380
   tagttttgga ggaattccag taccaggaaa acctgggacc tttttaaaga aaaacttcca 1440
   tggatgcatc gaaaaccttt actacaatgg agtaaacata attgacctgg ctaagagacg 1500
   aaagcatcag atctatactg tgggcaatgt cactttttcc tgctccgaac cacagattgt 1560
   gcccatcaca tttgtcaact ccagcggcag ctatttgctg ctgcccggca ccccccaaat 1620
   tgatgggctc tcagtgagtt tccagtttcg aacatggaac aaggatggtc tgcttctgtc 1680
   cacagagctg tctgagggct cgggaaccct gctgctgagc ctggagggtg gaatcctgag 1740
   actcgtgatt cagaaaatga cagaacgcgt agctgaaatc ctcacaggca gcaacttgaa 1800
   tgatggcctg tggcactcgg ttagcatcaa cgccaggagg aaccgcatca cgctcactct 1860
   ggatgatgaa gcagcacccc cggctccaga cagcacttgg gtgcagattt attctggaaa   1920
   tagctactat tttggagggt gccccgacaa tctcaccgat tcccaatgtt taaatcccat   1980
   taaggctttc caaggctgca tgaggctcat ctttattgat aaccagccca aggacctcat   2040
   ttcagttcag caaggttccc tggggaattt tagtgattta cacattgatc tgtgtagcat   2100
   caaagacagg tgtttgccaa actactgtga acatggagga agctgctccc agtcctggac   2160
   taccttctat tgtaactgca gtgacacaag ttacactggt gccacctgcc acaactccat   2220
   ctacgagcaa tcctgcgagg tgtacaggca ccaggggaat acagccggct tcttctacat   2280
   cgactcagat ggcagcggcc cactgggacc tctccaggtg tactgcaata tcactgagga   2340
   caagatctgg acatcagtgc agcacaacaa tacagagctg acccgagtgc ggggcgctaa   2400
   ccctgagaag ccctatgcca tggccttgga ctacgggggc agcatggaac agctggaggc   2460
   cgtgatcgac ggctctgagc actgtgagca ggaggtggcc taccactgca ggaggtcccg   2520
   cctgctcaac acgccggatg gaacaccatt tacctggtgg attgggcggt ccaatgaaag   2580
   gcacccttac tggggaggtt cccctcctgg ggtccagcag tgtgagtgtg gcctagacga   2640
   gagctgcctg gacattcagc acttttgcaa ttgcgacgct gacaaggatg aatggacaaa   2700
   tgatactggc tttctttcct tcaaagacca cttgcctgtc actcagatag ttatcactga   2760
   taccgacaga tcaaactcag aagccgcttg gagaattggt cccttgcgtt gctatggtga   2820
   ccgacgcttc tggaacgccg tctcatttta tacagaagcc tcttacctcc actttcctac   2880
   cttccatgcg gaattcagtg ccgatatttc cttctttttt aaaaccacag cattatccgg   2940
   agttttccta gaaaatcttg gcattaaaga cttcattcga ctcgaaataa gctctccttc   3000
   agagatcacc tttgccatcg atgttgggaa tggtcctgtg gagcttgtag tccagtctcc   3060
   ttctcttctg aatgacaacc aatggcacta tgtccgggct gagaggaacc tcaaggagac   3120
   ctccctgcag gtggacaacc ttccaaggag caccagggag acgtcggagg agggccattt   3180
   tcgactgcag ctgaacagcc agttgtttgt agggggaacg tcatccagac agaaaggctt   3240
   cctaggatgc attcgctcct tacacttgaa tggacagaaa atggacctgg aagagagggc   3300
   aaaggtcaca tctggagtca ggccaggctg ccccggccac tgcagcagct acggcagcat   3360
   ctgccacaac gggggcaagt gtgtggagaa gcacaatggc tacctgtgtg attgcaccaa   3420
   ttcaccttat gaagggccct tttgcaaaaa agaggtttct gctgtttttg aggctggcac   3480
   gtcggttact tacatgtttc aagaacccta tcctgtgacc aagaatataa gcctctcatc   3540
   ctcagctatt tacacagatt cagctccatc caaggaaaac attgcactta gctttgtgac   3600
   aacccaggca cccagtcttt tgctctttat caattcttct tctcaggact tcgtggttgt   3660
   tctgctctgc aagaatggaa gcttacaggt tcgctatcac ctaaacaagg aagaaaccca   3720
   tgtattcacc attgatgcag ataactttgc taacagaagg atgcaccact tgaagattaa   3780
   ccgagaggga agagagctta ccattcagat ggaccagcaa cttcgactca gttataactt   3840
   ctctccggaa gtagagttca gggttataag gtcactcacc ttgggcaaag tcacagagaa   3900
   tcttggtttg gattctgaag ttgctaaagc aaatgccatg ggttttgctg gatgcatgtc   3960
   ttccgtccag tacaaccaca tagcaccact gaaggctgcc ctgcgccatg ccactgtcgc   4020
   gcctgtgact gtccatggga ccttgacgga atccagctgt ggcttcatgg tggactcaga   4080
   tgtgaatgca gtgaccacgg tgcattcttc atcagatcct tttgggaaga cagatgagcg   4140
   ggaaccactc acaaatgctg ttcgaagtga ttcggcagtc atcggagggg tgatagcagt   4200
   ggtgatattc atcatcttct gtatcatcgg catcatgacc cggttcctct accagcacaa   4260
   gcagtcacat cgtacgagcc agatgaagga gaaggaatat ccagaaaatt tggacagttc   4320
   cttcagaaat gaaattgact tgcaaaacac agtgagcgag tgtaaacggg aatatttcat   4380
   ctgagaaact gcagggttcc tactactctt ttttcttgtt gttcaattat ctcctccccc   4440
   tcttctctcc tgtcttttga tttggtcatt ctctttattt tctgcttgcc atgtcttttc   4500
   tggaacatac ttgcatccac cacagcatca attcccttga tccagcccaa gagaccaggc   4560
   agccatggcc actgccttcc tctctgatga acctatcggg tgaaaacgac cactcaagag   4620
   actgacttcg ccattcaaga caaggaagag acacatgtgt gcactcctgc atgttcagtt   4680
   ctgtacttcc agtttctaaa atgcactgtt cagttttcca accacttggt ggttcaggct   4740
   tgctttgaac ctgagctctt aggcacatga cggtcattcc tgacatcctc cccagctcaa   4800
   gtctattctt accatagaac ccagggcagg gagagaagaa cctagaggcc tggtttgctt   4860
   tggtggcat                                                           4869